(19) Europäisches Patentamt
European Patent Office
Office européen des brevets

(11) Publication number: **0 184 384 B1**

(12) # EUROPEAN PATENT SPECIFICATION

(45) Date of publication of patent specification: **02.08.89**

(51) Int. Cl.⁴: **C 07 D 215/56,**
C 07 D 498/06, C 07 F 7/10,
A 61 K 31/47, A 61 K 31/535

(21) Application number: **85308651.0**

(22) Date of filing: **28.11.85**

(54) **Substituted dihydroquinolone carboxylic acids, anti-bacterial compositions containing them.**

(30) Priority: **06.12.84 US 679146**
**06.12.84 US 679150**

(43) Date of publication of application:
**11.06.86 Bulletin 86/24**

(45) Publication of the grant of the patent:
**02.08.89 Bulletin 89/31**

(84) Designated Contracting States:
**AT BE CH DE FR GB IT LI LU NL SE**

(56) References cited:
**GB-A-2 015 513**
**GB-A-2 094 305**
**US-A-3 472 859**
**US-A-3 960 868**
**US-A-4 382 892**

**The file contains technical information submitted after the application was filed and not included in this specification**

(73) Proprietor: **PFIZER INC.**
**235 East 42nd Street**
**New York, N.Y. 10017 (US)**

(72) Inventor: **Gilligan, Paul J.**
**699, Orange Street**
**New Haven Connecticut (US)**
Inventor: **Witty, Michael J.**
**13, Kingston Close**
**River Dover Kent (GB)**
Inventor: **McGuirk, Paul R.**
**22, Lincoln Drive**
**Gales Ferry Connecticut (US)**

(74) Representative: **Moore, James William, Dr.**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 184 384 B1

**Description**

This invention relates to 1-substituted-6-fluoro-7-aryl-(8-fluoro)-1,4-dihydro-quinol-4-one 3-carboxylic acids and their esters and cation salts, their preparation, and antibacterial compositions containing these compounds.

Since the introduction of nalidixic acids in 1963, a considerable number of patents and scientific papers have been published on analogs of this compound. Representative of these publications is U.S. Patent No. 3,472,859 granted October 14, 1969 disclosing *inter alia* compounds of the following formula

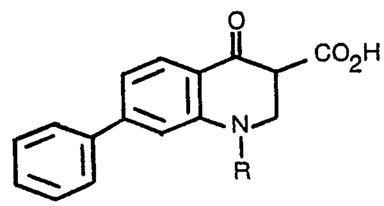

wherein R is lower alkyl. Example 7 of the patent discloses fungistatic activity for the compound wherein R is methyl, but no antibacterial data are provided.

United Kingdom Patent Application GB 2094305A describes 7-[4-(p-aminobenzyl)-1-piperazinyl]-1-ethyl-6,8-difluoro-1,4-dihydro-4-oxoquinoline-3-carboxylic. acid as an antibacterial agent. Patent Application EP 0117473A describes substituted piperazinyl derivatives of 1-cyclopropyl-1,4-dihydro-4-oxo-quinoline-3-carboxylic acid which may be substituted at the 6-position by halogen or nitro.

The compounds of the present invention are 1,4-dihydroquinol-4-one-3-carboxylic acids of the formula

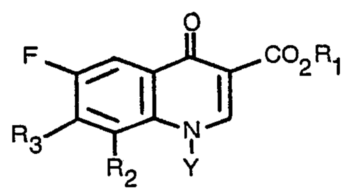

I

wherein

$R_1$ is hydrogen, alkyl of 1 to 6 carbon atoms, benzyl or a pharmaceutically acceptable cation;

$R_2$ is hydrogen or fluoro;

Y is alkyl, haloalkyl or polyhaloalkyl of 1 to 3 carbon atoms, hydroxyethyl, cyclopropyl, vinyl, allyl, phenyl, 4-hydroxyphenyl or 4-fluorophenyl; and $R_3$ is phenyl substituted by R' and R'', wherein R' is hydrogen, alkylsulfinyl of 1 to 4 carbon atoms alkylsulfonyl of 1 to 4 carbon atoms, hydroxy, alkoxy of 1 to 4 carbon atoms, hydroxyalkyl of 1 to 3 carbon atoms, amino, aminoalkyl of 1 to 3 carbon atoms, formamido, alkanoylamino of 2 or 3 carbon atoms, aminosulfonyl, nitro, formyl, or N-(N',N'-dimethylformamidino); and R'' is hydrogen, 3-hydroxy or 3-chloro.

Preferred compounds are those wherein $R_1$ is hydrogen, or a pharmaceutically acceptable cation, and those wherein Y is ethyl.

Specific preferred compounds of the invention in which $R_2$ is fluorine are

6,8-difluoro-7-phenyl-1-ethyl-1,4-dihydroquinol-4-one 3-carboxylic acid
6,8-difluoro-7-(4-hydroxyphenyl)-1-ethyl-1,4-dihydroquinol-4-one 3-carboxylic acid
6,8-difluoro-7-(3-chloro-4-hydroxyphenyl)-1-ethyl-1,4-dihydroquinol-4-one 3-carboxylic acid
6,8-difluoro-7-(3-hydroxyphenyl)-1-ethyl-1,4-dihydro-quinol-4-one 3-carboxylic acid
6,8-difluoro-7-(4-aminomethylphenyl)-1-ethyl-1,4-dihydroquinol-4-one 3-carboxylic acid
6,8-difluoro-7-(3-aminomethylphenyl)-1-ethyl-1,4-dihydroquinol-4-one 3-carboxylic acid
6,8-difluoro-7-(4-hydroxymethylphenyl)-1-ethyl-1,4-dihydroquinol-4-one 3-carboxylic acid
6,8-difluoro-7-(3-hydroxymethylphenyl)-1-ethyl-1,4-dihydroquinol-4-one 3-carboxylic acid
6,8-difluoro-7-(4-methylsulfinylphenyl)-1-ethyl-1,4-dihydroquinol-4-one 3-carboxylic acid
6,8-difluoro-7-(4-methoxyphenyl)-1-ethyl-1,4-dihydro-quinol-4-one 3-carboxylic acid
6,8-difluoro-7-(3-methylsulfonylphenyl)-1-ethyl-1,4-dihydroquinol-4-one 3-carboxylic acid
6,8-difluoro-7-(4-hydroxyphenyl)-1-methyl-1,4-dihydroquinol-4-one 3-carboxylic acid and
6,8-difluoro-7-(3-hydroxymethyl-4-hydroxy)-1-ethyl-1,4-dihydroquinol-4-one 3-carboxylic acid.

Specific preferred compounds wherein $R_2$ is hydrogen are

6-fluoro-7-phenyl-1-ethyl-1,4-dihydroquinol-4-one 3-carboxylic acid
6-fluoro-7-(4-aminophenyl)-1-ethyl-1,4-dihydroquinol-4-one 3-carboxylic acid
6-fluoro-7-(4-nitrophenyl)-1-ethyl-1,4-dihydroquinol-4-one 3-carboxylic acid
6-fluoro-7-(4-formamidophenyl)-1-ethyl-1,4-dihydroquinol-4-one 3-carboxylic acid
6-fluoro-7-(4-acetamidophenyl)-1-ethyl-1,4-dihydroquinol-4-one 3-carboxylic acid
6-fluoro-7-(4-(N-(N'N'-dimethylformamidino))phenyl)-1-ethyl-1,4-dihydroquinol-4-one 3-carboxylic acid

6-fluoro-7-(4-formylphenyl)-1-ethyl-1,4-dihydroquinol-4-one 3-carboxylic acid
6-fluoro-7-(4-hydroxymethylphenyl)-1-ethyl-1,4-dihydroquinol-4-one 3-carboxylic acid
6-fluoro-7-(3-hydroxymethylphenyl)-1-ethyl-1,4-dihydroquinol-4-one 3-carboxylic acid
6-fluoro-7-(4-aminosulfonylphenyl)-1-ethyl-1,4-dihydroquinol-4-one 3-carboxylic acid and
6-fluoro-7-(4-hydroxyphenyl-1-(4-fluorophenyl-1,4-dihydroquinol-4-one carboxylic acid.

The present invention also relates to antibacterial compositions comprising an antibacterially acceptable carrier and a compound of formula I. Preferred compositions are those wherein the compound of formula I is a preferred compound as described above.

The term "polyhaloalkyl" when used in the definition of $R_3$ means an alkyl having more than one halogen. Examples of such polyhaloalkyl groups are dichloromethyl, dibromoethyl, perfluoropropyl.

The term "halo" or "halogen" whenever used in the claims and the description, e.g. in the definition of $R_3$, means fluoro, chloro, bromo or iodo.

The compounds of formula I may be prepared by transition metal catalyst coupling of an appropriate arylmetallic compound containing group $R_3$ as defined above with the appropriate 7-$R_5$-quinolinone ester of the formula II

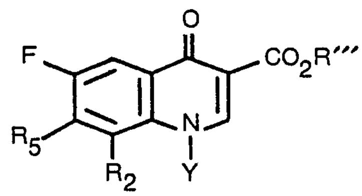

II

wherein $R_2$ and Y are as defined above, R''' is alkyl of 1 to 6 carbon atoms and $R_5$ is bromo or iodo.

The coupling reaction is carried out in a reaction-inert solvent, i.e. a solvent which does not interfere with the coupling reaction of the arylmetallic compound with the compound of formula II. Suitable reaction inert solvents are ethers, e.g. dialkylethers such as diethylether and dipropylether, dimethoxyethane, and cyclic ethers such as tetrahydrofuran (THF). Cosolvents may be used with the ethers, if desired, e.g. to enhance the solubility therein of the reaction materials. Examples of suitable cosolvents are aliphatic and aromatic hydrocarbons of 5 to 10 carbon atoms such as benzene and toluene. Other suitable cosolvents are cosolvent complexing agents such as tetramethylethylenediamine (TMEDA) and hexamethylphosphonictriamide (HMPA) as known to those skilled in the art.

The arylmetallic compounds containing group $R_3$ may be prepared by known methods. For instance, they may be prepared from the corresponding halide of formula $R_3R_5$ in which $R_3$ and $R_5$ are as defined above by direct lithium-halogen exchange using n-butyl, sec-butyl or t-butyl lithium followed by transmetallation by a wide variety of salts by known methods such as described by E. Negishi, Organometallics in Organic Synthesis, Vol. 1, page 104. The salts used in the transmetallation may be selected from the salts of zinc, cadmium, magnesium, mercury, tin, silver, copper, boron and aluminum, preferably zinc. The most commonly used salts are the halides, particularly chlorides, bromides, and iodides, and cyanides such as copper cyanide. The most advantageous salt is zinc chloride.

The above treatment with a butyl lithium compound is generally carried out at −78 to −50°C, in a suitable solvent such as ethers alone or in admixture with an aliphatic or aromatic hydrocarbon solvent having from 5 to 10 carbon atoms. Suitable ethers are for instance dialkylethers such as diethylether, cyclic ethers such as THF, and dimethoxyethane. Suitable hydrocarbon solvents are for instance toluene and benzene. The treatment with the butyl lithium is best carried out in THF at −78°C.

The arylmetallic compounds may also be prepared by direct reaction of the corresponding arylhalide with a metal in the zerovalent state. These metals are alkali metals such as lithium, sodium and potassium, alkaline earth metals such as magnesium, and transition metals such as zinc. The reaction temperatures may range from −100°C for very active metals to 100°C. Again, solvents of use in this process are ethers alone or in admixture with hydrocarbons having from 5 to 10 carbon atoms such that at least one equivalent of the ether is present.

Alternatively, the arylmetallic compounds may be formed by hydrogen-metal exchange between a corresponding aryl compound and a strong base such as t-butoxide-butyl-lithium, TMEDA-butyllithium, or lithium or potassium hexamethyldisilizane.

The arylmetallic compound is generally not isolated before reaction by coupling with compound (II).

The coupling of the arylmetallic compound with the 7-haloquinolinone ester of formula II is in the presence of a transition metal catalyst usually in an amount of 0.5—10 mole%. The transition metal catalyst may be of the 0 or II oxidation state and contains a transition metal, that is a metal selected from Groups Ib through VIIIb and VIII of the Periodic Table as set out in Lange's Handbook of Chemistry, 11th Edition. Suitable metals are for instance platinum, cobalt, iron, zirconium, molybdenum, ruthenium, manganese and rhodium, and preferred metals are palladium, platinum and nickel.

The transition metal is combined with a ligand to form the catalyst. Suitable known ligands are phosphorus-containing ligands such as $PPh_3$, $P(CH_3)_3$, $P(C_2H_5)_3$, P(o-tolyl)$_3$, P(o-xylyl)$_3$, $Ph_2P(CH_2)_n$ $PPh_2$ wherein n is an integer from 1 to 4, and $(CH_3)_2$ $P(CH_2)_2$ $P(CH_3)_2$; or cis $Ph_2CH=CHPh_2$, acetonylacetonate, 2,2'-bipyridyl pyridine and others as described in Tamao et al., Bull. Chem. Soc. Japan, 49(7), 1959 (1976).

Other transition metal catalysts are disclosed in A. Sekiya et al., J. Organometal. Chem., 118, 349—354 (1976) and E. Negishi, Acc. Chem. Res., 15, 340—348 (1982) and references cited therein. Ph is phenyl.

The preferred transition metal catalysts are $(PPh_3)_2$ $NiHal_2$, $(PPh_3)_4$ Ni, $(PPh_3)_2$ $PdHal_2$, and $(PPh_3)_4$ Pd wherein Hal is chloro, bromo or iodo and Ph is phenyl. The most preferred catalyst is tetrakis(triphenylphosphine) palladium.

The reaction temperature of the coupling process generally ranges from room temperature to 50°C.

Methods for the preparation of the compounds of formula II are analogous to those described in the art. The overall reactions of two methods analgous to those in the prior art are set out in reaction Schemes A and B hereafter.

In Scheme A, an aniline of formula IV wherein $R_2$ and $R_5$ are as defined above is reacted with a dialkyl alkoxymethylene malonate of formula V wherein R''' is an alkyl group of 1 to 6 carbon atoms or benzyl. The reaction is generally carried out without solvent at about 100 to 200°C, preferably 150 to 175°C, for about 0.5 to 24 hours, usually for 0.5 to 2 hours. The resulting intermediates of formula VI are crystallized from a hydrocarbon or ethereal solvent such as light petroleum or diethyl ether and cyclized by heating at about 150 to 250°C in high boiling solvents such as dichlorobenzene, tetralin, diphenylether or diethyleneglycol dimethylether, preferably Dowtherm A (Registered Trade Mark) which is a commercially available high boiling solvent mixture of diphenylether and dibenzofuran. The reaction time ranges from about 0.5 to 12 hours.

The intermediates of formula VII formed above are N-substituted with a halide Y-Hal wherein Y is as defined above and Hal is halogen. Examples of suitable halides are ethyliodide, 2-fluoro-1-iodoethane, allylbromide and 2-bromoethanol. The adduct formed on reaction with 2-bromoethanol may be converted to a compound of formula I wherein Y is vinyl by hydroxyl activation with e.g. thionyl chloride followed by elimination with a suitable base such as triethylamine, diazabicycloundecene and diazabicyclononane. Generally, the substitution is carried out in DMF with an inorganic base such as potassium carbonate temperatures ranging from room temperature to 110°C. This reaction similarly proceeds when $R_5$ in formula VII is replaced by $R_3$ as defined before to produce the compounds of formula I provided that R''' in compounds (VII) is $R_1$ as defined above except $R_1$ is hydrogen requiring an additional hydrolysis step.

The mono- or di-fluoro anilines of formula IV, also used in the reaction of Scheme B hereafter, may be prepared by conventional nitration and reduction methods from the corresponding fluorobenzenes which are commercially available.

SCHEME A

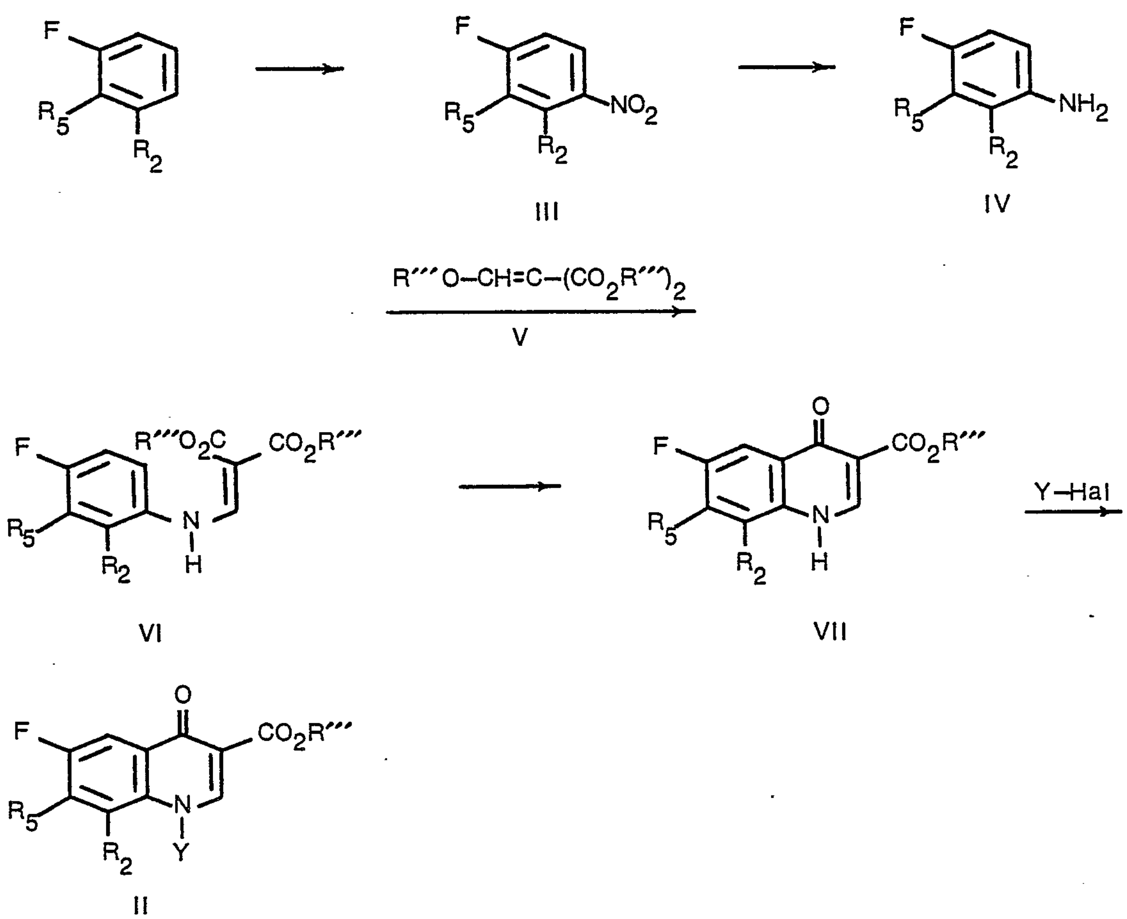

In Scheme B, an aniline of formula X wherein $R_2$, $R_5$ and Y are as defined above is reacted with a dialkyl alkoxymethylene malonate of formula V wherein each R''' is alkyl having 1 to 6 carbon atoms or benzyl. The reaction conditions are as described above with reference to the conversion of compounds IV into V in Scheme A.

The cyclization of compound XI formed above is by heating in an acidic medium such as polyphosphoric acid at about 100 to 250°C for about 0.5 to 24 hours, preferably at 100 to 150°C for 0.5 to 2 hours. This procedure is described by Albrecht, R., Prog. Drug Res., Vol. 21, 35—49 (1977). The resulting ester of formula II is usually purified by recrystallization or chromatography.

The compounds of formula X may be prepared from those of formula IV by conventional methods. For instance, in Scheme B, an aniline of formula IV may be reacted with acetic anhydride in ethanol at about 25 to 100°C. The formed compound of formula VIII is reacted with a suitable base such as sodium hydride and N-substituted with an appropriate halide, tosylate or mesylate containing group Y. The acetyl group in the formed compound of formula IX is removed by refluxing in aqueous medium such as 6N hydrochloric acid to form the compound of formula X.

Alternatively, N-substituted anilines of formula X may be formed by reductive amination with an appropriate aldehyde and a suitable reducing agent such as diborane, palladium on carbon with hydrogen, sodium borohydride or sodium cyanoborohydride

SCHEME B

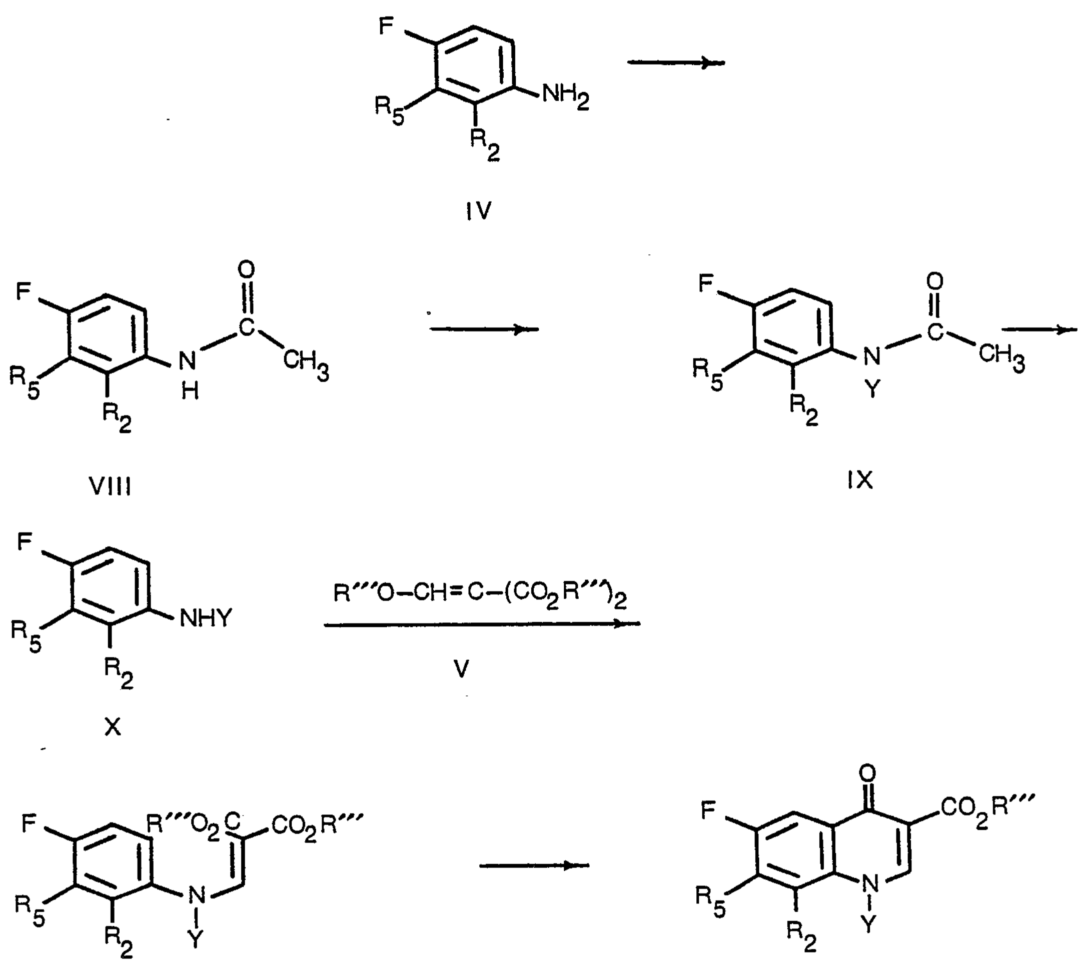


IV

VIII

IX

X

XI

II

or by acylation of said aniline of formula IV by reaction with the appropriate anhydride or acid chloride and direct reduction to anilines X with diborane in THF.

The initial N-substitution of formula IV in Scheme B, rather than the later one of compounds of formula VII in Scheme A, is particularly useful when Y is polyfluoroalkyl since substitution with polyfluoroalkyl halides is not a viable route.

Compounds of formula I wherein $R_3$ is phenyl substituted by alkylsulfinyl or alkylsulfonyl may be obtained by conventional oxidation of the corresponding compounds wherein $R_3$ is phenylmercaptoalkyl. The compounds (I) wherein $R_3$ is hydroxyphenyl may be formed by reacting the corresponding compound

wherein $R_3$ is methoxyphenyl with an ether-cleaving reagent such as borontribromide or trimethylsilyliodide.

Compounds (I) wherein $R_3$ is hydroxymethylphenyl are conveniently prepared by reacting the corresponding compound wherein $R_3$ is trialkylsilyloxymethylphenyl wherein each alkyl has 1 to 4 carbon atoms with an ether-cleaving agent as described above. Compounds (I) wherein $R_3$ is nitrophenyl may be prepared by conventional nitration of compounds (I) wherein $R_3$ is phenyl. Reduction of such nitrophenyl compounds provides compounds wherein $R_3$ is aminophenyl.

Compounds (I) wherein $R_3$ is N,N-dimethylformamidophenyl may be formed by reacting compounds wherein $R_3$ is aminophenyl with formic acid or a formic acid derivative such as ethylformate. Compounds (I) wherein $R_3$ is alkanoylamino may be formed by reacting corresponding compounds wherein $R_3$ is aminophenyl with an alkanoyl group containing reagent such as an acid chloride or anhydride, e.g. acetic anhydride.

Compounds (I) wherein $R_3$ is formamidinophenyl may be formed by reacting compounds (I) wherein $R_3$ is amino-phenyl with dimethylformamide dimethylacetal in toluene.

Aminosulfonyl-substituted phenyl compounds may be obtained by reacting compounds (I) wherein $R_3$ is chlorosulfonylphenyl with an amino group introducing reagent such as ammonia or an amine. The chlorosulfonyl group is introduced by reacting compounds wherein $R_3$ is phenyl with chlorosulfonic acid. Alkylation of the aminosulfonylphenyl compounds will result in compounds (I) wherein $R_3$ is alkylaminosulfonylphenyl or dialkylaminosulfonylphenyl. These latter compounds alternatively may be obtained by reacting compounds (I) wherein $R_3$ is chlorosulfonylphenyl with a monoalkyl amine or a dialkyl amine, respectively.

Compounds (I) wherein $R_3$ is formylphenyl may be prepared by oxidation of the corresponding compound wherein $R_3$ is hydroxymethylphenyl, e.g. by oxidation with oxalyl chloride in DMSO and triethylamine.

Compounds (I) wherein $R_1$ is hydrogen may be obtained by acid or base hydrolysis of the corresponding esters wherein $R_1$ is alkyl or benzyl, or by hydrogenolysis of corresponding compounds wherein $R_1$ is benzyl.

The compounds of formula I have antibacterial properties particularly when in the acid, cation salt or acid addition salt form.

The pharmaceutically acceptable cation salts of the compounds of formula I may be prepared by conventional methods. For instance, the salts may be prepared by treating the compound of formula I in which $R_1$ is hydrogen with an aqueous solution of the desired pharmaceutically acceptable cation and evaporating the resulting solution to dryness, preferably under reduced pressure. Suitable pharmaceutically acceptable cation salts for this purpose include alkali metal salts such as potassium, sodium and lithium salts, alkaline earth metal salts such as calcium and magnesium salts, and the ammonium or organic amine salts, such as choline or diethanolamine salts.

The compounds of the invention can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules either alone or in admixture with excipients, or in the form of elixirs or suspensions containing flavoring or coloring agents. In the case of animals, they are advantageously contained in an animal feed or drinking water in a concentration of 10—1000 ppm, preferably 10—300 ppm. They can be injected parenterally, for example, intramuscularly, intravenously or subcutaneously. For parenteral administration, they are best used in the form of a sterile aqueous solution which can contain other solutes, for example, enough salt or glucose to make the solution isotonic. Generally, compound (I) is dissolved in a pharmaceutically acceptable liquid such as water which may contain buffers, preservatives, materials to make the solution isotonic, e.g. isotonic saline, or other materials known to those skilled in art. The reconstituted solution so prepared may be used for direct parenteral injection or may be added to a solution such as an I.V. solution for slow administration by infusion.

The invention also provides pharmaceutical compositions comprising an antibacterially effective amount of a compound of the formula (I) together with a pharmaceutically acceptable diluent or carrier.

The compounds of the invention can be administered to humans for the treatment of bacterial diseases by either the oral or parenteral routes, and may be administered orally at dosage levels of about 0.1 to 500 mg/kg/day, advantageously 0.5—200 mg/kg/day, and parenterally at dosage levels of about 50 to 500 mg/kg/day given in a single dose or up to 3 divided doses. Variations will necessarily occur depending on the weight and condition of the subject being treated and the particular route of administration chosen as will be known to those skilled in the art.

The antibacterial activity of the compounds of the invention is shown by testing according to the Steer's replicator technique which is a standard *in vitro* bacterial testing method described by E. Steers et al., Antibiotics and Chemotherapy, 9, 307(1959).

Example 1

3-Bromo-2,4-difluoronitrobenzene

A. A mixture of 10 ml concentrated $HNO_3$ and 10 ml concentrated $H_2SO_4$ was added dropwise with

cooling to a stirred solution of 30 g 2,6-difluorobromobenzene in 50 ml of concentrated sulfuric acid. The rate of addition was controlled such that the temperature of the mixture did not rise above 55°C. After the addition was complete the reaction mixture was allowed to stir at room temperature for 2 hours. It was then poured onto ice and the resulting solid was collected by filtration, then poured onto ice and the resulting solid was collected by filtration, washed with water and dried, yielding a pale yellow solid, m.p. 50—51.5°C, 84% yield (31.2 g).

| | | | | | |
|---|---|---|---|---|---|
| Anal.: Calcd. for $C_6H_2BrF_2NO_2$: | C, 30.28; | H, 0.85; | Br, 33.57; | F, 15.96; | N, 5.89%. |
| Found: | C, 30.34; | H, 0.98; | Br, 33.81; | F, 16.04; | N, 5.81%. |

3-Amino-2,6-difluorobromobenzene

B. A mixture of 31.2 g of 3-bromo-2,4-difluoronitrobenzene, 150 ml concentrated HCl and 150 g stannous chloride dihydrate was placed in a preheated oil bath at 60°C. A small quantity of diethyl ether was then added to bring about solution. After heating at 60°C for 30 minutes, the mixture was cooled and then poured into 1500 ml ice/water. The solution was then basified to pH 13 using 30% aqueous sodium hydroxide while maintaining the temperature below 25°C by external cooling. The mixture was extracted three times with chloroform and the combined organic extracts were washed with water. The organic phase was dried and evaporated to yield a white solid, m.p. 77—78°C, yield 90.5% (24.7 g).

Ethyl 7-bromo-6,8-difluoro-4-hydroxyquinoline-3-carboxylate

C. A stirred mixture of 26 g of 3-amino-2,6-difluorobromobenzene and 27 g of diethyl ethoxymethylenemalonate was heated at 150°C for 1 hour. After cooling, 100 ml of Dowtherm "A" (commercially available high boiling inert solvent mixture of diphenylether and dibenzofuran) was added and the mixture was heated under nitrogen at 260°C for 1.5 hour. The mixture was cooled to room temperature and 200 ml of hexanes was added. The resulting precipitate was collected by filtration, washed with hexanes and dried to give a cream solid, m.p. 285°C, yield 78% (32.32 g).

Ethyl 7-bromo-6,8-difluoro-1-ethyl-1,4-dihydroquinol-4-one 3-carboxylate

D. A mixture of 32 g of ethyl 7-bromo-6,8-difluoro-4-hydroxyquinoline-3-carboxylate, 45.1 g iodoethane and 50 g of anhydrous potassium carbonate in 450 ml N,N-dimethylformamide was heated at 90°C with stirring for 2 hours. The mixture was then cooled, poured into water and the resulting precipitate was collected by filtration and dried. This crude product was purified by eluting on a silica column with ethyl acetate/hexanes (60:40) which on isolation yielded white crystals, m.p. 153—155°C, yield 86% (29.84 g).

| | | | |
|---|---|---|---|
| Analysis: Calcd. for $C_{14}H_{12}BrF_2NO_3$: | C, 46.66; | H, 3.33; | N, 3.88%. |
| Found: | C, 46.38; | H, 3.37; | N, 3.87%. |

Ethyl 6,8-difluoro-1-ethyl-7-phenyl-1,4-dihydroquinol-4-one 3-carboxylate ($R_1$ = ethyl, $R_2$ = F, $R_3$ = phenyl, Y = ethyl)

E. A 2.7 M solution (1 ml) of phenyllithium in 60/30 cyclohexane/diethyl ether was diluted with 10 ml dry tetrahydrofuran. This mixture was stirred under nitrogen at room temperature and a solution of 409 mg anhydrous zinc chloride in 10 ml tetrahydrofuran was added. After 20 minutes, a solution of 360 mg of ethyl 7-bromo-6,8-difluoro-1-ethyl-1,4-dihydroquinol-4-one 3-carboxylate in 10 ml tetrahydrofuran and 124 mg of palladium tetrakis(triphenyl phosphine) were added. The resulting mixture was heated at 40—45°C for 18 hours. The mixture was then cooled and poured into 200 ml water. The mixture was stirred for 20 minutes and then extracted three times with chloroform. The organic extracts were combined, washed with water, dried and evaporated to give the crude product. This material was purified by eluting on a silica column with ethyl acetate which on isolation yielded the pure product as a solid (251 mg, 70% yield). NMR ($CDCl_3$, 60MHz): 8.4 (s, 1H), 8.1 (dd, 1H, J=9Hz and 2Hz), 7.5 (s, 5H), 4.4 (m, 4H), 1.5 (t, 3H, J=6.5Hz), 1.4 (t, 3H, 6.5Hz).

MS: Calcd. for $C_{20}H_{17}F_2NO_3$: 357.1178; Found: 357.1164.

6,8-Difluoro-1-ethyl-7-phenyl-1,4-dihydroquinol-4-one 3-carboxylic acid ($R_1$ = H; $R_2$ = F; $R_3$ = phenyl; Y = ethyl).

F. Ethyl 6,8-difluoro-1-ethyl-7-phenyl-1,4-dihydroquinol-4-one 3-carboxylate (186 mg) was dissolved in 10 ml 1:4 hydrochloric acid/acetic acid and the mixture was heated at reflux for 3.5 hours. The mixture was cooled and the resulting precipitate collected by filtration, washed with water and dried, yielding solid product, m.p. >260°C (114 mg, 66% yield). NMR (DMSO-$d_6$, 250MHz): 8.75 (s, 1H), 7.9 (dd, 1H, J=9Hz and 2Hz), 7.35 (s, 5H), 4.5 (m, 2H), 1.35 (t, 3H, J=6.5Hz). Ms: Calcd. for $C_{18}H_{13}F_2NO_3$: 329.0864. Found: 329.0860.

| | | | |
|---|---|---|---|
| Anal.: Calcd. for $C_{18}H_{13}F_2NO_3 \cdot 2/3H_2O$: | C, 63.34; | H, 4.00; | N, 4.10%. |
| Found: | C, 63.08; | H, 4.02; | N, 4.14%. |

### Example 2

Ethyl 6,8-difluoro-1-ethyl-7-(4-methylthiophenyl)-1,4-dihydroquinol-4-one 3-carboxylate ($R_1$ = ethyl; $R_2$ = F; $R_3$ = 4-methylthiophenyl; Y = ethyl)

12.5 Ml of a 2 M solution of t-butyllithium in pentane was added dropwise to a stirred solution of 2.53 g

4-bromothioanisole in 35 ml of dry tetrahydrofuran at −78°C under nitrogen. After stirring for 1 hour at −78°C, a solution of 1.7 g anhydrous zinc chloride in 25 ml of tetrahydrofuran was added and the mixture was stirred for a further 15 minutes at −78°C. To the resulting solution, 620 mg of palladium tetrakis(triphenylphosphine) was added, followed by the slow addition of a solution of 1.8 g of ethyl 7-bromo-6,8-difluoro-1-ethyl-1,4-dihydroquinol-4-one 3-carboxylate in 10 ml tetrahydrofuran. After the addition was complete, the mixture was stirred for a further 15 minutes at −78°C and then allowed to warm slowly to room temperature. After a further 24 hours, 5 ml of 5% aqueous ammonium chloride was added followed by 5 ml of 1 M hydrochloric acid. The mixture was stirred for 10 minutes and it was then poured into 200 ml water and extracted with three 50 ml portions of chloroform. The combined organic extracts were washed once with water, dried and evaporated to yield the crude product as a yellow solid. This was purified by eluting on a silica column with ethyl acetate which on isolation gave the required product (1.7 g, yield 55%). NMR ($CDCl_3$, 60MHz); 8.35 (s, 1H), 8.05 (dd, 1H, J=9Hz and 2Hz), 7.35 (m, 4H), 4.35 (2q, 4H, J=6.5Hz), 2.6 (s, 3H), 1.5 (t, 3H, J=6.5Hz), 1.4 (t, 3H, J=6.5Hz).

MS: Calcd. for $C_{21}H_{15}F_2NO_3S$: 403.1054; Found: 403.1031.

## Example 3

Ethyl 6,8-difluoro-1-ethyl-7-(4-methylsulfinylphenyl)-1,4-dihydroquinol-4-one 3-carboxylate ($R_1$ = ethyl; $R_2$ = F, $R_3$ = 4-methylsulfinylphenyl; Y = ethyl)

A. A solution of 101 mg 85% m-chloroperbenzoic acid in 3 ml dichloromethane was added dropwise to a stirred solution of 201 mg of ethyl 6,8-difluoro-1-ethyl-7-(4-methylthiophenyl)-1,4-dihydroquinol-4-one 3-carboxylate in dichloromethane at room temperature. After 1.5 hour, the reaction mixture was washed twice with 10 ml 5% aqueous sodium bicarbonate and the organic phase was dried and evaporated to give the crude required product as an oil. This was purified by elution on silica gel, first with ethyl acetate and then using a gradient elution with ethyl acetate/methanol. The product was a hard glass (90 mg, yield 43%). NMR ($CDCl_3$, 60MHz): 8.4 (s, 1H), 8.1 (dd, 1H, J=9Hz and 2Hz), 7.7 (m, 4H), 4.4 (2q, 4H, J=6.5Hz), 2.8 (s, 3H), 1.5 (t, 3H, J=6.5Hz), 1.4 (t, 3H, J=6.5Hz).

6,8-Difluoro-1-ethyl-7-(4-methylsulfinylphenyl)-1,4-dihydroquinol-4-one 3-carboxylic acid ($R_1$ = H; $R_2$ = F; $R_3$ = 4-methylsulfinylphenyl; Y = ethyl)

B. 60 Mg (72% yield) was prepared from 90 mg ethyl 6,8-difluoro-1-ethyl-7-(4-methylsulfinylphenyl)-1,4-dihydroquinol-4-one 3-carboxylate by the method of Example 1F. White solid was obtained, m.p. 260°C. NMR (DMSO $d_6$, 250MHz); 9.07 (s, 1H), 8.1 (dd, 1H, J=9Hz and 2Hz), 7.87 (m, 4H), 4.65 (m, 2H), 2.85 (s, 3H), 1.5 (t, 3H, J=6Hz).

MS: Calcd. for $C_{19}H_{15}F_2NO_4S$: 391.0690. Found: 391.0621.

| | | | |
|---|---|---|---|
| Anal.: Calcd. for $C_{19}H_{15}F_2NO_4S$: | C, 58.31; | H, 3.83; | N, 3.58%. |
| Found: | C, 57.89; | H, 4.15; | N, 3.36%. |

## Example 4

4-Fluoro-3-phenylnitrobenzene

A. 2-Fluoro-5-nitroaniline (30 g) was added portionwise over 45 minutes to a refluxing solution of 60 ml isoamyl nitrite in 225 ml benzene. After heating for a further 30 minutes at reflux, the mixture was cooled and the solvent removed by evaporation in vacuo. The residue was triturated many times with hot petroleum ether and the combined extracts were evaporated to give the product as a red oil (19.9 g, yield 49%). This material was used without further purification. NMR ($CDCl_3$, 60MHz): 8.0—8.4 (m, 3H), 7.1—7.6 (m, 5H).

4-Fluoro-3-phenylaniline

B. A mixture of 18.5 g of 4-fluoro-3-phenylnitrobenzene, 76.7 g of stannous chloride dihydrate and 150 ml concentrated hydrochloric acid was heated at 60°C for 2 hours with stirring. After cooling, the resulting precipitate was dissolved in water and the mixture was basified with sodium carbonate. The mixture was extracted five times with chloroform and the combined organic extracts were dried and evaporated to yield the product as a pale brown oil (10.9 g, yield 68%) which was used without further purification. NMR ($CDCl_3$, 60MHz): 7.15 (m, 5H), 7.1—6.1 (m, 3H), 3.3 (br,s, 2H).

Diethyl 4-fluoro-3-phenylanilinomethylenemalonate

C. A mixture of 10.9 g of 4-fluoro-3-phenylaniline and 12.54 g diethyl ethoxymethylenemalonate was heated at 150°C for 15 minutes. The mixture was then cooled and triturated with petroleum ether to give the solid product (16.73 g, 80% yield) which was used without further purification. NMR ($CDCl_3$, 60MHz): 8.3 (d, 1H, J=14Hz), 7.5—6.9 (m, 8H) 4.2 (m, 4H), 1.4 (m, 6H).

Ethyl 6-fluoro-7-phenylquinolin-4-ol 3-carboxylate

D. A solution of 16.73 g of diethyl 4-fluoro-3-phenylanilinomethylenemalonate in 100 ml Dowtherm "A" was heated at 250°C with stirring for 2.5 hours. The mixture was cooled and the resulting precipitate was collected by filtration, washed with petroleum ether and dried, m.p. >270°C. (12.37 g, 85% yield). NMR (trifluoroacetic acid-d, 60MHz); 9.3 (s, 1H), 8.2 (m, 2H), 7.5 (m, 5H), 4.7 (q, 2H, J=6.5Hz), 1.5 (t, 3H, J=6.5Hz).

Ethyl 1-ethyl-6-fluoro-7-phenyl-1,4-dihydroquinol-4-one 3-carboxylate ($R_1$ = ethyl; $R_2$ = H; $R_3$ = phenyl; Y = ethyl)

E. A mixture of 12.4 g ethyl 6-fluoro-7-phenylquinolin-4-ol 3-carboxylate, 11 g anhydrous potassium carbonate and 18.7 g iodoethane in 100 ml N,N-dimethyl formamide was heated at 80°C for 4 hours. The reaction mixture was cooled, poured into ethyl acetate and the mixture was extracted several times with water. The organic phase was dried and evaporated to give a solid product which was washed well with diethyl ether and dried. White solid was obtained, m.p. 180—182°C (9.16 g, 68% yield). NMR ($CDCl_3$, 60MHz); 8.4 (s, 1H), 8.05 (d, 1H, J=10Hz), 7.5 (m, 6H), 4.3 (q, 2H, J=6.5Hz), 4.25 (q, 2H, J=6.5Hz), 1.55 (t, 3H, J=6.5Hz), 1.35 (t, 3H, J=6.5Hz).

1-Ethyl-6-fluoro-7-phenyl-1,4-dihydroquinol-4-one 3-carboxylic acid ($R_1$ = $R_2$ = H; $R_3$ = phenyl; Y = ethyl).

F. A mixture of 0.44 g ethyl 1-ethyl-6-fluoro-7-phenyl-1,4-dihydroquinol-4-one 3-carboxylate and 10 ml 1 M sodium hydroxide was stirred at 90°C for 1 hour. The mixture was cooled and poured into 20 ml water. The pH was adjusted to 7 with 1 M hydrochloric acid and the resulting precipitate was collected by filtration, washed with water, then ether, and dried, yielding a white solid, m.p. 253—256°C (0.26 g, 65% yield). NMR (Trifluoroacetic acid-d, 60MHz): 9.4 (s, 1H), 8.3 (m, 2H), 7.6 (m, 5H), 4.9 (q, 2H, J=6.5Hz), 1.8 (t, 3H, J=6.5Hz).

| | | | |
|---|---|---|---|
| Anal.: Calcd for $C_{18}H_{14}FNO_3 \cdot 1.5H_2O$: | C, 63.90; | H, 5.02; | N, 4.14%. |
| Found: | C, 63.52; | H, 4.66; | N, 3.92%. |

Example 5

Ethyl 1-ethyl-6-fluoro-7-(4-nitrophenyl)-1,4-dihydroquinol-4-one 3-carboxylate ($R_1$ = ethyl; $R_2$ = H; $R_3$ = 4-nitrophenyl; Y = ethyl)

A. Ethyl 1-ethyl-6-fluoro-7-phenyl-1,4-dihydroquinol-4-one 3-carboxylate (4 g) was added portionwise to a stirred mixture of 12 ml concentrated sulphuric acid and 12 ml concentrated nitric acid at 0°C. After 1 hour at 0°C, the mixture was poured onto ice and the resulting precipitate was collected by filtration. This solid was washed with ethyl acetate and then recrystallized from hot ethyl acetate to give a solid, m.p. 210—212°C (2.88 g, 68% yield). NMR (Trifluoroacetic acid d, 250MHz): 9.5 (s, 1H), 8.5 (m, 4H), 8.0 (m, 2H), 5.05 (q, 2H, J=6.5Hz), 4.75 (q, 2H, J=6.5Hz), 1.85 (t, 3H, J=6.5Hz), 1.5 (t, 3H, J=6.5Hz). MS: 384 (parent), 312 (base peak).

1-Ethyl-6-fluoro-7-(4-nitrophenyl)-1,4-dihydroquinol-4-one 3-carboxylic acid ($R_1$ = H; $R_2$, $R_3$ and Y as in Example 5A)

B. A mixture of 0.65 g of ethyl 1-ethyl-6-fluoro-7-(4-nitrophenyl)-1,4-dihydroquinol-4-one 3-carboxylate and 15 ml 1 M sodium hydroxide was heated at 90°C with stirring for 30 minutes. The mixture was cooled and then poured into 40 ml water. The pH of the mixture was adjusted to 7 with 1 M hydrochloric acid and the resulting precipitate was collected by filtration. The solid was washed with water, ether and ethyl acetate and then it was recrystallized from hot N,N-dimethylformamide to give a white solid, m.p. >270°C (0.29 g, 48% yield). NMR (Trifluoroacetic acid-d, 60MHz): 9.5 (s, 1H), 8.5—7.7 (m, 6H), 5.1 (q, 2H), 1.85 (t, 3H).

Example 6

Ethyl 1-ethyl-6-fluoro-7-(4-aminophenyl)-1,4-dihydroquinol-4-one 3-carboxylate ($R_1$ = Y = ethyl; $R_2$ = H; $R_3$ = 4-aminophenyl

A. A mixture of 1.7 g of ethyl 1-ethyl-6-fluoro-7-(4-nitrophenyl)-1,4-dihydroquinol-4-one 3-carboxylate, 12.75 g stannous chloride dihydrate and 13 ml concentrated hydrochloric acid were stirred at 0°C for 15 minutes and then at room temperature for 2 hours. The mixture was cooled and the resulting precipitate was collected by filtration, dissolved in water and the solution was adjusted to pH 9 with solid sodium bicarbonate. It was then extracted several times with ethyl acetate and then chloroform. The combined organic extracts were washed with water, dried and evaporated to give a white solid, m.p. 248—250°C (1.25 g, 78% yield). NMR (DMSO-$d_6$, 250MHz); 8.7 (s, 1H), 7.9 (d, 1H, J=11.5Hz), 7.75 (d, 1H, J=6Hz), 7.4 (dd, 2H, J=9Hz), 6.7 (d, 2H, J=9Hz), 4.5 (q, 2H, J=6.5Hz), 4.25 (q, 2H, J=6.5Hz), 1.45 (t, 3H, J=6.5Hz), 1.3 (t, 3H, J=6.5Hz).

1-Ethyl-6-fluoro-7-(4-aminophenyl)-1,4-dihydroquinol-4-one 3-carboxylic acid ($R_1$ = $R_2$ = H; $R_3$ = 4-aminophenyl; Y = ethyl)

B. A mixture of 0.8 g ethyl 1-ethyl-6-fluoro-7-(4-aminophenyl)-1,4-dihydroquinol-4-one 3-carboxylate and 16 ml 2 M hydrochloric acid were heated at reflux for 6 hours. The mixture was cooled and the resulting precipitate was washed with water and dried to give the product as a yellow solid, m.p. 270—272°C (0.59 g, 78% yield). NMR (Trifluoroacetic acid-d, 250MHz): 9.55 (s, 1H), 8.6 (d, 1H, J=8.5Hz), 8.45 (d, 1H, J=6Hz), 8.0 (d, 2H, J=9.5Hz), 7.85 (d, 2H, J=9.5Hz), 5.1 (m, 2H), 1.9 (t, 3H). MS: 326 (parent), 282 (base peak).

| | | | |
|---|---|---|---|
| Anal.: Calcd. for $C_{18}H_{15}FN_2O_2 \cdot 2.5H_2O$: | C, 58.22; | H, 5.39; | N, 7.54%. |
| Found: | C, 58.25; | H, 4.73; | N, 7.44%. |

Example 7

1-Ethyl-6-fluoro-7-(4-formamidophenyl)-1,4-dihydroquinol-4-one 3-carboxylic acid ($R_1$ = $R_2$ = H; $R_3$ = 4-formamidophenyl; Y = ethyl)

1-Ethyl-6-fluoro-7-(4-aminophenyl)-1,4-dihydroquinol-4-one 3-carboxylic acid (100 mg) in 1 ml formic acid was heated at reflux for 5 hours. The mixture was cooled and the solvent removed by evaporation in vacuo. The residual solid was washed with water and diethyl ether and dried, yielding the product as a pale yellow solid, m.p. >270°C. (86 mg, 79% yield). NMR (DMSO-$d_6$/Trifluoroacetic acid-d, 250MHz): 9.4 (s, 1H), 8.5 (s, 0.5H), 8.4 (d, 1H, J=9Hz), 8.3 (d, 1H, J=6Hz), 8.2 (s, 0.5H), 7.9 (d, 2H, J=8Hz), 7.8 (d, 2H, J=8Hz), 5.0 (q, 2H, J=6.5Hz), 1.75 (t, 3H, J=6.5Hz).

| | | | |
|---|---|---|---|
| Anal.: Calcd. for $C_{19}H_{15}FN_2O_4 \cdot 1.5H_2O$: | C, 59.84; | H, 4.72; | N, 7.35%. |
| Found: | C, 60.24; | H, 4.31; | N, 7.08%. |

Example 8

1-Ethyl-6-fluoro-7-(4-acetamidophenyl)-1,4-dihydroquinol-4-one 3-carboxylic acid ($R_1$ = $R_2$ = H; $R_3$ = 4-acetamidophenyl; Y = ethyl)

1-Ethyl-6-fluoro-7-(4-aminophenyl)-1,4-dihydroquinol-4-one 3-carboxylic acid (50 mg) was placed in a mixture of 1 ml acetic acid and 0.5 ml acetic anhydride and the mixture was stirred at room temperature for 1.5 hours. The resulting precipitate was collected by filtration washed with water and ether and dried, yielding the product as a white solid of m.p. >250°C (35 mg, 62% yield). NMR (Trifluoroacetic acid-d, 250MHz): 9.5 (s, 1H), 8.55 (d, 1H, J=9Hz), 8.4 (d, 1H, J=6Hz), 7.85 (d, 2H, J=6Hz), 7.75 (d, 2H, J=6Hz), 5.05 (m, 2H), 2.55 (s, 3H), 1.85 (t, 3H, J=6Hz).

Example 9

1-Ethyl-6-fluoro-7-(N-(N',N'-dimethylformamidinophenyl)-1,4-dihydroquinol-4-one 3-carboxylic acid ($R_1$ = $R_2$ = H; $R_3$ = N-(N',N'-dimethylformamidinophenyl); Y = ethyl)

A mixture of 1-ethyl-6-fluoro-7-(4-aminophenyl)-1,4-dihydroquinol-4-one 3-carboxylic acid (100 mg) and N,N-dimethylformamide dimethylacetal (74 mg) in toluene was heated at reflux for 3 hours. A further 38 mg of N,N-dimethylformamide dimethylacetal was then added and the mixture was heated at reflux for a further 2 hours. The mixture was cooled, the solvent removed by evaporation in vacuo and the residual solid was washed with ether and dried, yielding the product as a pale yellow solid of m.p. 215—218°C (70 mg, 57% yield). NMR (DMSO-$d_6$/Trifluoroacetic acid-d, 250MHz): 9.2 (s, 1H), 8.5 (s, 1H), 8.3 (d, 1H, J=9Hz), 8.15 (d, 1H, J=6Hz), 7.85 (d, 2H, J=6Hz), 7.6 (d, 2H, J=6Hz). 4.8 (m, 2H), 3.45 (d, 6H, J=18Hz), 1.65 (t, 3H, J=6Hz). MS: 381 (66%).

| | | | |
|---|---|---|---|
| Anal.: Calcd. for $C_{21}H_{20}FN_3O_3$: | C, 63.16; | H, 5.51; | N, 10.52%. |
| Found: | C, 62.94; | H, 5.25; | N, 9.94%. |

Example 10

Ethyl 1-ethyl-6-fluoro-7-(4-chlorosulfonylphenyl)-1,4-dihydroquinol-4-one 3-carboxylate

A. Chlorosulfonic acid (10 ml) was slowly added over 15 minutes to a stirred solution of ethyl 1-ethyl-6-fluoro-7-phenyl-1,4-dihydroquinol-4-one 3-carboxylate (2.5 g) in dichloromethane (20 ml) at 0°C. The mixture was stirred for a further 15 minutes at 0°C and then at room temperature for 1.5 hour. The mixture was poured into ice/water and the mixture was extracted twice with ethyl acetate. The combined organic extracts were dried and evaporated to give the product as an oily solid (1.4 g, 42% yield) which was used immediately without further purification. NMR ($CDCl_3$, 60MHz): 8.6 (s, 1H), 8.4—7.4 (m, 6H), 4.4 (2q, 4H, J=6.5Hz), 1.6 (t, 3H, J=6.5Hz), 1.4 (t, 3H, J=6.5Hz).

Ethyl 1-ethyl-6-fluoro-7-(4-aminosulfonylphenyl)-1,4-dihydroquinol-4-one 3-carboxylate ($R_1$ = ethyl; $R_2$ = H; $R_3$ = 4-aminosulfonylphenyl; Y = ethyl)

B. A saturated ethanolic ammonia solution (1 ml) was added to a stirred solution of ethyl 1-ethyl-6-fluoro-7-(4-chlorosulfonylphenyl)-1,4-dihydroquinol-4-one 3-carboxylate (0.7 g) in 8 ml dichloromethane at −50°C. The mixture was allowed to warm slowly to room temperature. After 2 hours, the mixture was poured into water and the resulting precipitate was collected by filtration, washed with water, ethyl acetate and ether and dried, yielding the product as a white solid (0.4 g, 60% yield). NMR (DMSO-$d_6$, 60MHz): 8.7 (s, 1H), 8.2—7.4 (m, 6H), 4.5 (q, 2H, J=6.5Hz), 4.3 (q, 2H, J=6.5Hz), 1.5 (t, 3H, J=6.5Hz), 1.4 (t, 3H, J=6.5Hz).

1-Ethyl-6-fluoro-7-(4-aminosulfonylphenyl)-1,4-dihydroquinol-4-one 3-carboxylic acid ($R_1$ = H; $R_2$, $R_3$ and Y as in Example 10B)

C. A mixture of 0.4 g ethyl 1-ethyl-6-fluoro-7-(4-aminosulfonylphenyl)-1,4-dihydroquinol-4-one 3-carboxylate, 5 ml ethanol and 5 ml 1 M sodium hydroxide was heated at 90°C for 30 minutes. The mixture was cooled, then concentrated in vacuo and the pH was adjusted to 7 with 1 M hydrochloric acid. The resulting precipitate was collected by filtration, washed and dried to give the product as a white solid, m.p. >270°C (0.3 g, 78% yield). NMR (DMSO-$d_6$/Trifluoroacetic acid d, 250MHz): 9.45 (s, 1H), 8.5 (d, 1H, J=9Hz), 8.4 (d, 1H, J=6Hz), 8.2 (d, 2H, J=6.5Hz), 7.95 (d, 2H, J=6.5Hz), 5.0 (q, 2H), 1.8 (t, 3H, J=6.5Hz).

| | | | |
|---|---|---|---|
| Anal.: Calcd. for $C_{18}H_{15}FN_2O_5S \cdot 0.5H_2O$: | C, 54.10; | H, 4.01; | N, 7.01%. |
| Found: | C, 53.59; | H, 4.00; | N, 6.71%. |

Example 11

Ethyl 6,8-Difluoro-7-(3-methoxyphenyl)-1-ethyl-1,4-dihydroquinol-4-one 3-carboxylate

A. 471 mg (60% yield) was prepared by the method of Example 2 using 935 mg of 1-bromo-3-methoxybenzene, 5 ml 2 M t-butyl lithium solution, 818 mg anhydrous zinc chloride, 720 mg ethyl 7-bromo-6,8-difluoro-1-ethyl-1,4-dihydroquinol-4-one 3-carboxylate and 248 mg tetrakis(triphenylphosphine) palladium. NMR ($CDCl_3$, 60MHz): 8.4 (s, 1H), 8.1 (dd, 1H, J=9 and 2Hz), 7.6—6.9 (m, 4H), 4.4 (2q, 4H, J=6.5Hz), 3.8 (s, 3H), 1.5 (t, 3H, J=6.5Hz), 1.4 (t, 3H, J=6.5Hz).

MS: Calcd. for $C_{21}H_{19}F_2NO_4$: 387.1285. Found: 387.1267.

6,8-Difluoro-7-(3-methoxyphenyl)-1-ethyl-1,4-dihydroquinol-4-one 3-carboxylic acid

B. 327 mg (76% yield) was prepared by the method of Example 1F from 471 mg ethyl 6,8-difluoro-7-(3-methoxyphenyl)-1-ethyl-1,4-dihydroquinol-4-one 3-carboxylate and recovered as a white solid of m.p. >260°C NMR (DMSO-$d_6$, 250MHz: 9.05 (s, 1H), 8.05 (dd, 1H, J=9Hz and 2Hz), 7.5 (t, 1H), 7.15 (m, 2H), 4.65 (m, 2H), 3.85 (s, 3H), 1.45 (t, 3H, J=6Hz).

MS: Calcd. for $C_{19}H_{15}F_2NO_4$: 359.0963. Found: 359.0979.

| | | | |
|---|---|---|---|
| Anal.: Calcd. for $C_{19}H_{15}F_2NO_4$: | C, 63.50; | H, 4.17; | N, 3.89%. |
| Found: | C, 63.28; | H, 4.14; | N, 3.85%. |

Example 12

6,8-Difluoro-7-(3-hydroxyphenyl)-1-ethyl-1,4-dihydroquinol-4-one 3-carboxylic acid

A solution of 292 mg 6,8-difluoro-7-(3-methoxyphenyl)-1-ethyl-1,4-dihydroquinol-4-one 3-carboxylic acid in 50 ml dichloromethane was stirred at −78°C while 8.1 ml of 1 M boron tribromide in dichloromethane was added dropwise. The reaction mixture was then allowed to warm slowly to room temperature and it was stirred overnight. The mixture was cooled to 5°C and quenched with saturated aqueous sodium bicarbonate. The mixture was filtered and the filter residue was extracted with hot tetrahydrofuran. The organic extracts were evaporated to give a solid residue which was treated with 2 M hydrochloric acid. The resulting solid was collected by filtration, washed with water and dried, yielding the required product as a white solid of m.p. >260°C (217 mg, 77% yield). NMR (DMSO-$d_6$, 250MHz): 9.8 (s, 1H), 9.05 (s, 1H), 8.05 (dd, 1H, J=9Hz and 2Hz), 7.35 (t, 1H), 6.85 (m, 2H), 4.65 (m, 2H), 3.85 (s, 3H), 1.45 (t, 3H, J=6Hz).

MS: Calcd. for $C_{18}H_{13}F_2NO_4$: 345.0913. Found: 345.0856.

| | | | |
|---|---|---|---|
| Anal.: Calcd. for $C_{18}H_{13}F_2NO_4 \cdot 0.5H_2O$: | C, 61.01; | H, 3.95; | N, 3.95%. |
| Found: | C, 61.27; | H, 3.95; | N, 3.90%. |

Example 13

Ethyl 6,8-Difluoro-7-(4-methoxyphenyl)-1-ethyl-1,4-dihydroquinol-4-one 3-carboxylate

A. 7.7 g of crude product was prepared by the method of Example 2 using 4.67 g of 1-bromo-4-methoxybenzene, 25 ml 2M t-butyl lithium solution, 3.75 g anhydrous zinc chloride, 7.2 g ethyl 7-bromo-6,8-difluoro-1-ethyl-1,4-dihydroquinol-4-one 3-carboxylate and 1.5 g tetrakis(triphenylphosphine) palladium. A pale yellow solid was recovered of m.p. 171—172°C. NMR ($CDCl_3$, 60MHz): 8.3 (s, 1H), 8.0 (dd, 1H, J=9Hz and 2Hz), 7.6—6.9 (m, 4H), 4.4 (2q, 4H, J=6.5Hz), 3.8 (s, 3H), 1.5 (t, 3H, J=6.5Hz), 1.4 (t, 3H, J=6.5Hz).

MS: Calcd. for $C_{21}H_{19}F_2NO_4$: 387.1279. Found: 387.1277.

6,8-Difluoro-7-(4-methoxyphenyl)-1-ethyl-1,4-dihydroquinol-4-one 3-carboxylic acid

B. A mixture of 7.7 g of crude ethyl 6,8-difluoro-7-(4-methoxyphenyl)-1-ethyl-1,4-dihydroquinol-4-one 3-carboxylate, 50 ml tetrahydrofuran and 100 ml 1M hydrochloric acid was heated at reflux overnight. The reaction mixture was cooled and the resulting precipitate was collected by filtration, yielding the required product as a white solid (5.38 g, 75% yield) of m.p. 250—252°C. NMR (Trifluoroacetic acid-d, 60MHz): 9.2 (s, 1H), 8.1 (dd, 1H, J=9Hz and 2Hz), 7.4 (d, 2H, J=9Hz), 7.0 (d, 2H, J=9Hz), 4.8 (m, 2H), 3.9 (s, 3H), 1.6 (t, 3H, J=6.5Hz).

MS: Calcd. for $C_{18}H_{15}F_2NO_4$: 359.0973. Found: 359.0986. Anal.: Calcd. for $C_{18}H_{15}F_2NO_4.0.5H_2O$: C, 61.96; H, 4.35; N, 3.80%. Found: C, 61.78; H, 4.40; N, 3.60%.

Example 14

6,8-Difluoro-7-(4-hydroxyphenyl)-1-ethyl-1,4-dihydroquinol-4-one 3-carboxylic acid

A solution of 312 mg 6,8-difluoro-7-(4-methoxyphenyl)-1-ethyl-1,4-dihydroquinol-4-one 3-carboxylic acid in 40 ml dichloromethane was stirred at −78°C while 8.69 ml of 1M boron tribromide in dichloromethane was added dropwise. The reaction mixture was then allowed to warm slowly to room temperature and it was stirred overnight. The mixture was cooled to 5°C and quenched with saturated aqueous sodium bicarbonate. The mixture was extracted with dichloromethane and then several times with a mixture of tetrahydrofuran and ethyl acetate. The latter extracts were evaporated and the resulting solid was stirred with 1M hydrochloric acid for 30 minutes and the product was collected by filtration, washed with water and dried. The required product was a white solid (264 mg, 88% yield) of m.p. >270°C.

NMR (DMSO-$d_6$, 250 MHz): 9.05 (s, 1H), 8.0 (d, 1H, J=9Hz), 7.4 (d, 2H, J=9Hz), 6.95 (d, 2H, J=9Hz), 4.65 (m, 2H), 1.45 (t, 3H, J=6Hz).

MS: Calcd. for $C_{18}H_{13}F_2NO_4$: 345.0813. Found 345.0813. Anal.: Calcd. for $C_{18}H_{13}F_2NO_4.H_2O$: C, 59.50; H, 4.13; N, 3.85%. Found: C, 59.59; H, 4.07; N, 3.74%.

Example 15

4-Bromobenzyl dimethyl-t-butylsilyl ether

A. A mixture of 18.7 g 4-bromobenzyl alcohol, imidazole (13.6 g), dimethyl-t-butylsilyl chloride (15 g) and N,N-dimethylformamide (100 ml) was stirred at room temperature for 2 hours. The reaction mixture was poured into water and extracted three times with ether. The combined organic extracts was washed with water, brine and dried. Evaporation gave the product as a colorless oil (29.8 g, 99% yield) which was used without further purification. NMR ($CDCl_3$, 250MHz): 7.55 (d, 2H, J=9Hz), 7.25 (d, 2H, J=9Hz), 4.7 (s, 2H), 0.9 (s, 9H), 0.0 (s, 6H).

Anal.: Calcd. for $C_{13}H_{21}BrOSi$: C, 51.83; H, 6.97%. Found: C, 51.48; H, 6.93%.

Ethyl 1-ethyl-6,8-difluoro-7-(4-(dimethyl-t-butylsilyloxymethyl)-phenyl)-1,4-dihydroquinol-4-one 3-carboxylate

B. 5.1 g (51% yield) was prepared by the method of Example 2 from 7.53 g of 4-bromobenzyl dimethyl-t-butylsilyl ether, 25 ml 2M t-butyl lithium solution, 3.75 g anhydrous zinc chloride, 7.2 g ethyl 7-bromo-6,8-difluoro-1-ethyl-1,4-dihydroquinol-4-one 3-carboxylate and 1.5 g tetrakis(triphenylphosphine) palladium. The title compound formed had a m.p. of 143—144°C. NMR (DMSO-$d_6$, 250MHz): 8.7 (s, 1H), 7.95 (dd, 1H, J=9 and 2Hz), 7.5 (q, 4H), 4.85 (s, 2H), 4.5 (br, m, 2H), 4.25 (q, 2H), 1.45 (t, 3H, J=7Hz), 1.4 (t, 3H, J=7Hz), 0.95 (s, 9H), 0.15 (s, 6H).

Anal.: Calcd. for $C_{27}H_{33}F_2NO_4Si$: C, 64.67; H, 6.58; N, 2.79%. Found: C, 64.75; H, 6.72; N, 2.78%.

Ethyl 1-ethyl-6,8-difluoro-7-(4-hydroxymethylphenyl)-1,4-dihydroquinol-4-one 3-carboxylate

C. A mixture of 5.1 g ethyl 1-ethyl-6,8-difluoro-7-(4-(dimethyl-t-butylsilyloxymethyl)-phenyl)-1,4-dihydroquinol-4-one 3-carboxylate and 10.2 ml of 1M tetra-n-butylammonium fluoride in tetrahydrofuran was stirred at room temperature for 2 hours. The reaction mixture was poured into water and extracted three times with ether and once with chloroform. The combined organic extracts were dried and evaporated to yield the product as a yellowish solid of m.p. 230—232°C (3.8 g, 96% yield). NMR (DMSO-$d_6$, 250MHz): 8.7 (s, 1H), 7.9 (dd, 1H, J=2Hz and 10Hz), 7.5 (s, 4H), 4.6 (d, 2H), 4.5 (m, 2H), 4.25 (q, 2H, J=6.5Hz), 1.45 (t, 3H, J=6.5Hz), 1.35 (t, 3H, J=6.5Hz).

MS: Calcd. for $C_{21}H_{19}F_2NO_4$: 387.1279. Found 387.1275.

1-Ethyl-6,8-difluoro-7-(4-hydroxymethylphenyl)-1,4-dihydroquinol-4-one 3-carboxylic acid

D. 600 mg (43% yield) was prepared by the method of Example 13B from 1.5 g ethyl 1-ethyl-6,8-difluoro-7-(4-hydroxymethylphenyl)-1,4-dihydroquinol-4-one 3-carboxylate as a white solid of m.p. 269°C. NMR (DMSO-$d_6$, 250MHz): 9.05 (s, 1H), 8.05 (dd, 1H, J=2Hz and 9Hz), 7.55 (s, 4H), 5.35 (t, 1H, J=4Hz), 4.65 (m, 2H), 4.6 (d, 2H, J=4Hz), 1.45 (t, 3H, J=6Hz).

MS: Calcd. for $C_{19}H_{15}F_2NO_4$: 359.0965. Found: 359.0972. Anal.: Calcd. for $C_{19}H_{15}F_2NO_4$: C, 63.51; H, 4.18; N, 3.90%. Found: C, 63.15; H, 4.21; N, 3.66%.

Example 16

3-Bromobenzyl dimethyl-t-butylsilyl ether

A. A mixture of 5.0 g 3-bromobenzyl alcohol, imidazole (3.64 g), dimethyl-t-butylchlorosilane (4.02 g) and N,N-dimethylformamide (25 ml) was stirred at room temperature for 2 hours. The reaction mixture was poured into water and extracted three times with ether. The combined organic extract was washed with water, brine and dried. Evaporation gave the product as a colorless oil (6.83 g, 85% yield) which was used without further purification.

Ethyl 1-ethyl-6,8-difluoro-7-(3-(dimethyl-t-butylsilyloxymethyl)-phenyl)-1,4-dihydroquinol-4-one 3-carboxylate

B. 7.0 g (87% yield) was prepared by the method of Example 2 from 6.82 of 3-bromobenzyl dimethyl-t-butylsilyl ether 25 ml 2M t-butyl lithium solution, 3.4 g anhydrous zinc chloride, 5.9 g ethyl 7-bromo-6,8-difluoro-1-ethyl-1,4-dihydroquinol-4-one 3-carboxylate and 1.5 g tetrakis(triphenylphosphine) palladium. The melting point was 107—109°C. NMR (DMSO-$d_6$, 250MHz): 8.7 (s, 1H), 7.92 (dd, 1H, J=9 and 2Hz), 7.49 (m, 4H), 4.82 (s, 2H), 4.47 (m, 2H), 4.26 (q, 2H), 1.42 (t, 3H, J=7Hz), 1.3 (t, 3H, J=7Hz), 0.94 (s, 9H), 0.1 (s, 6H).

Anal.: Calcd. for $C_{27}H_{33}F_2NO_4Si$: C, 64.67; H, 6.58; N, 2.79%. Found: C, 64.51; H, 6.59; N, 2.75%.

Ethyl 1-ethyl-6,8-difluoro-7-(3-hydroxymethylphenyl)-1,4-dihydroquinol-4-one 3-carboxylate

C. 4.2 g (78% yield) was prepared by the method of Example 15C from 7.0 g of ethyl 1-ethyl-6,8-difluoro-7-(3-(dimethyl-t-butylsilyloxymethyl)-phenyl)-1,4-dihydroquinol-4-one 3-carboxylate and 14 ml of 1M tetra-n-butylammonium fluoride in tetrahydrofuran. A yellow solid of m.p. 162—166°C was recovered. NMR (DMSO-$d_6$, 250MHz): 8.7 (s, 1H), 7.95 (dd, 1H, J=2Hz and 9Hz), 7.5 (m, 4H), 5.3 (t, 3H, J=6Hz), 4.6 (d,

2H, J=6Hz), 4.5 (m, 2H), 4.25 (q, 2H, J=6.5Hz), 1.45 (t, 3H, J=6.5Hz), 1.3 (t, 3H, J=6.5Hz).

MS: Calcd. for $C_{21}H_{19}F_2NO_4$: 387.1279. Found: 387.1280.

1-Ethyl-6,8-difluoro-7-(3-hydroxymethylphenyl)-1,4-dihydroquinol-4-one 3-carboxylic acid

D. 330 mg (92% yield) was prepared by the method of Example 13B from 387 mg ethyl 1-ethyl-6,8-difluoro-7-(3-hydroxymethylphenyl)-1,4-dihydroquinol-4-one 3-carboxylate as a white solid of m.p. 230—231°C. NMR (DMSO-$d_6$, 250MHz): 9.1 (s, 1H), 8.05 (dd, 1H, J=9Hz and 2Hz), 7.5 (m, 4H), 4.65 (m, 2H), 4.6 (s, 2H), 1.45 (t, 3H, J=6Hz).

Anal.: Calcd. for $C_{19}H_{15}F_2NO_4.0.5H_2O$: C, 61.95; H, 4.35; N, 3.80%. Found: C, 61.89; H, 4.50; N, 3.48%.

Example 17

Ethyl 1-ethyl-6,8-difluoro-7-(4-azidomethylphenyl)-1,4-dihydroquinol-4-one 3-carboxylate

A. A solution of 585 mg methanesulfonyl chloride in 20 ml dichloromethane was added dropwise to a stirred solution of 15 ml triethylamine and 1.8 g ethyl 1-ethyl-6,8-difluoro-7-(4-hydroxymethylphenyl)-1,4-dihydroquinol-4-one 3-carboxylate in 100 ml dichloromethane at 0°C. After the addition was complete, the reaction mixture was extracted with 120 ml water and the organic phase was dried and evaporated to yield the mesylate product which was used directly without further purification.

This mesylate was dissolved in acetone containing 605 mg sodium azide. Water was added dropwise until solution of the azide was complete. The reaction mixture was stirred overnight at room temperature. The reaction mixture was partitioned between water and dichloromethane and the aqueous phase was extracted twice more and the combined organic extracts were washed with water and brine and dried. Evaporation gave the crude product which was purified by elution on silica gel yielding pure product as a solid of m.p. 149.5—150°C (850 mg, 44% yield). NMR (DMSO-$d_6$, 250MHz): 8.7 (s, 1H), 7.95 (dd, 1H, J=2Hz and 9Hz), 7.6 (ABq, 4H), 4.6 (s, 2H), 4.5 (m, 2H), 4.25 (q, 3H), J=6.5Hz), 1.45 (t, 3H, J=6.5Hz), 1.35 (t, 3H, J=6.5Hz).

MS: Calcd. for $C_{21}H_{18}F_2N_4O_3$: 412.1343. Found: 412.1344.

Anal.: Calcd. for $C_{21}H_{18}F_2N_4O_3$: C, 61.16; H, 4.37; N, 13.59%. Found: C, 61.65; H, 4.55; N, 13.13%.

Ethyl 1-ethyl-6,8-difluoro-7-(4-aminomethylphenyl)-1,4-dihydroquinol-4-one 3-carboxylate

B. A solution of 2.6 g ethyl 7-(4-azidomethylphenyl)-1-ethyl-6,8-difluoro-1,4-dihydroquinol-4-one 3-carboxylate in 50 ml methanol and 100 ml chloroform, containing 1.3 g 5% palladium on charcoal was hydrogenated at 12 p.s.i. for 2 hours. The reaction mixture was filtered and the filtrate was evaporated. The residue was triturated with a small quantity of chloroform and dried, yielding 2.2 g of the product as a solid (92% yield), m.p. 262—263°C. NMR (DMSO-$d_6$, 250MHz): 8.72 (s, 1H), 8.5 (br s, 2H), 7.95 (dd, 1H, J=2 and 9Hz), 7.65 (q, 4H), 4.49 (br m, 2H), 4.3 (q, 2H), 4.13 (s, 2H), 1.42 (t, 3H, J=7Hz), 1.3 (t, 3H, J=7Hz).

MS: Calcd. for $C_{21}H_{20}F_2N_2O_3$: 386.1437. Found: 386.1448.

1-Ethyl-6,8-difluoro-7-(4-aminomethylphenyl)-1,4-dihydroquinol-4-one 3-carboxylic acid hydrochloride salt

C. 135 mg (79.3% yield) was prepared by the method of Example 13B from 450 mg ethyl 7-(4-aminomethylphenyl)-1-ethyl-6,8-difluoro-1,4-dihydroquinol-4-one 3-carboxylate. White solid of m.p. >280°C. NMR (DMSO-$d_6$, 250MHz): 9.1 (s, 1H, 8.1 (dd, 1H, J=2Hz and 9Hz), 7.7 (ABqt, 4H), 4.65 (m, 2H), 4.15 (s, 2H), 1.45 (t, 3H, J=6.5Hz).

MS: Calcd. for $C_{19}H_{16}F_2N_2O_3$: 358.1129. Found: 358.1169.

Anal.: Calcd. for $C_{19}H_{16}F_2N_2O_3.HCl\cdot2H_2O$: C, 53.02; H, 4.88; N, 6.51%. Found: C, 53.66; H, 4.35; N, 6.60%.

Example 18

Ethyl 1-ethyl-6,8-difluoro-7-(3-azidomethylphenyl)-1,4-dihydroquinol-4-one 3-carboxylate

A. 2.28 g (54% yield) was prepared by the method of Example 17A using 1.3 g methanesulfonyl chloride in 25 ml dichloromethane, added to a solution of 25 ml triethylamine and 4.0 g ethyl 1-ethyl 6,8-difluoro-7-(3-hdyroxymethylphenyl)-1,4-dihydroquinol-4-one 3-carboxylate in 100 ml dichloromethane at 0°C, followed by 5.36 g sodium azide in 150 ml acetone. The product formed was a solid of m.p. 119°C. NMR (DMSO-$d_6$, 250MHz): 8.7 (s, 1H), 7.9 (dd, 1H, J=2Hz and 9Hz), 7.55 (m, 4H), 4.6 (s, 2H), 4.5 (m, 2H), 4.25 (q, 2H, J=6.5Hz), 1.45 (t, 3H, J=6.5Hz), 1.3 (t, 3H, J=6.5Hz).

MS: Calcd. for $C_{21}H_{18}F_2N_4O_3$: 412.1343. Found: 412.1363.

Anal.: Calcd. for $C_{21}H_{18}F_2N_4O_3$: C, 61.16; H, 4.37; N, 13.59%. Found: C, 61.27; H, 4.58; N, 12.77%.

Ethyl 1-ethyl-6,8-difluoro-7-(3-aminomethylphenyl)-1,4-dihydroquinol-4-one 3-carboxylate

B. 200 mg (85% yield) was prepared by the method of Example 17B from 250 mg ethyl 1-ethyl-6,8-difluoro-7-(3-azidomethylphenyl)-1,4-dihydroquinol-4-one 3-carboxylate. A pale brown solid was formed, m.p. 260—262°C. NMR (DMSO-$d_6$, 250MHz): 8.72 (s, 1H), 8.4 (br s, 2H), 7.97 (br d, 1H, J=9Hz), 7.65 (m, 4H), 4.49 (br m, 2H), 4.27 (q, 2H), 4.12 (s, 2H), 1.45 (t, 3H, J=7Hz), 1.32 (t, 3H, J=7Hz).

MS: 386 (parent), 314 (base).

1-Ethyl-6,8-difluoro-7-(3-aminomethylphenyl)-1,4-dihydroquinol-4-one 3-carboxylic acid hydrochloride salt

C. 135 mg (79.3% yield) was prepared by the method of Example 13B from 450 mg ethyl 1-ethyl-6,8-difluoro-7-(4-aminomethylphenyl-1,4-dihydroquinol-4-one 3-carboxylate. White solid of m.p. >280°C. NMR (DMSO-$d_6$, 250MHz): 9.1 (s, 1H), 8.1 (dd, 1H, J=2Hz and 9Hz), 7.65 (m, 4H), 4.65 (m, 2H), 4.1 (s, 2H), 1.45 (t, 3H, J=6.5Hz).

MS: Calcd. for $C_{19}H_{16}F_2N_2O_3$: 358.1129. Found: 358.1172. Anal.: Calcd. for $C_{19}H_{16}F_2N_2O_3.HCl.1.5H_2O$: C, 56.57; H, 4.46; N, 6.95%. Found: C, 56.80; H, 4.47; N, 6.99%.

Example 19

3-(2-Fluorophenyl)-toluene

A. A 1.46M solution (45 ml) of toluylmagnesium bromide in ether was added to a mixture of 10 g 2-bromofluorobenzene and 250 mg bis(diphenylphosphinoethane)-nickel dichloride in 70 ml dry diethyl ether. The mixture was then heated at reflux for 18 hours. The mixture was cooled to room temperature and quenched with 10% aqueous ammonium chloride, followed by 1M hydrochloric acid, and then partitioned between ether and water. The aqueous phase was extracted once more with ether and the combined organic extracts were dried and evaporated yielding the crude product as an oil which was purified by elution from silica gel with hexanes. The product was a colorless oil (7.0 g, 66% yield) which was used without further purification. NMR ($CDCl_3$, 60MHz): 7.4—6.9 (m, 8H), 2.4 (s, 3H).

3-(2-Fluorophenyl)-benzoic acid

B. A mixture of 7.0 g 3-(2-fluorophenyl)-toluene and 29.5 g potassium permanganate in 500 ml t-butanol and 100 ml water was heated at 90°C for 18 hours. The reaction mixture was cooled to room temperature, sufficient sodium bisulfite was added to dissolve the remaining potassium permanganate and manganese dioxide, and the pH was adjusted to 1 with hydrochloric acid. The resulting precipitate was collected by filtration, redissolved in ethyl acetate and this solution was dried and evaporated yielding the product as a white solid of m.p. 147—148°C after recrystallization from hexanes (5.7 g, 70% yield). NMR (DMSO-$d_6$, 250MHz): 8.2—7.3 (m, 8H).

MS: Calcd. for $C_{13}H_9F_2O$: 216.0585. Found: 216.0585.

3-(2-Fluoro-5-nitrophenyl)-benzoic acid

C. A solution of 420 mg potassium nitrate in 5 ml concentrated sulfuric acid was added dropwise to a stirred solution of 1 g 3-(2-fluorophenyl)-benzoic acid in 25 ml concentrated sulfuric acid at 0°C. The mixture was stirred at 0°C for 20 minutes and then poured into iced water. The resulting precipitate was extracted into ethyl acetate and the extracts were combined, dried and evaporated, yielding the product as a white solid (1.2 g, 100% yield), m.p. 272—274°C after recrystallization from ethyl acetate. NMR (DMSO-$d_6$, 250MHz): 8.4 (m, 2H), 8.16 (d, 1H J=2Hz), 8.05 (multiplets, 1H), 7.9 (multiplets, 1H), 7.66 (m, 2H), 3.4 (br s, 1H).

MS: Calcd. for $C_{13}H_8NFO_4$: 261.0438. Found: 261.0416.

Anal.: Calcd. for $C_{13}H_8NFO_4.1/4H_2O$: C, 58.75; H, 3.20; N, 5.27%. Found: C, 58.84; H, 3.19; N, 5.12%.

3-(2-Fluoro-5-nitrophenyl)-benzyl alcohol

D. A 1M solution (200 ml) of diborane in tetrahydrofuran was added to a stirred solution of 3-(2-fluoro-5-nitrophenyl)-benzoic acid (6.6 g) in 500 ml tetrahydrofuran. The reaction mixture was stirred at room temperature for 12 hours and then quenched with water and extracted with ethyl acetate. The organic extracts were dried and evaporated to yield a yellow solid which was purified by elution on silica gel with 40% ethyl acetate/hexanes. This gave the pure required product as a yellow solid (4.04 g, 65% yield), m.p. 113—114°C. NMR ($CDCl_3$, 250MHz): 8.42 (four line multiplet, 1H), 8.25 (8 line multiplet, 1H), 7.55 (m, 4H), 7.3 (four line multiplet, 1H), 4.8 (d, 2H, J=4.5Hz), 0.9 (t, 1H).

MS: Calcd. for $C_{13}H_{10}FNO_3$: 247.0645. Found: 247.0647.

Anal.: Calcd. for $C_{13}H_{10}FNO_3$: C, 63.16; H, 4.05; N, 5.67%. Found: C, 63.42; H, 4.11; N, 5.53%.

3-(3-t-Butyldimethylsilyloxymethylphenyl)-4-fluoronitrobenzene

E. A mixture of 4.04 g 3-(2-fluoro-5-nitrophenyl)-benzyl alcohol, 2.23 g imidazole and 2.46 g t-butyldimethylsilylchloride in 100 ml N,N-dimethylformamide was stirred at room temperature for 30 minutes. The reaction mixture was then partitioned between water and ether. The combined organic extracts were washed three times with water, dried and evaporated yielding the product as a yellow oil (5.9 g, 99% yield) which was purified on silica gel to give a yellow solid of m.p. 44—45°C. NMR ($CDCl_3$, 250MHz): 8.4 (dd, 1H), 8.34 (eight line multiplet, 1H), 7.5 (m, 4H), 7.3 (m, 1H), 4.83 (s, 2H), 0.95 (s, 9H), 0.15 (s, 6H).

Anal.: Calcd. for $C_{18}H_{21}FNO_3Si$: C, 63.16; H, 6.65; N, 3.88%. Found: C, 62.99; H, 6.62; N, 3.82%.

3-(3-t-Butyldimethylsilyloxymethylphenyl)-4-fluoroaniline

F. A solution of 5.92 g 3-(3-t-butyldimethylsilyloxymethylphenyl)-4-fluoronitrobenzene in 200 ml 50% ethyl acetate/hexanes was hydrogenated over 5 g of 5% palladium on carbon at 50 p.s.i. for 15 minutes. The reaction mixture was filtered and evaporated to yield the crude product (5.43 g, 100% yield) which was used directly without further purification. On purification, the compound is characterized by the following

data: NMR ($CDCl_3$, 250MHz): 7.45 (m, 4H), 6.98 (dd, 1H), 6.75 (dd, 1H), 6.62 (8 line multiplet, 1H), 4.8 (s, 2H), 3.6 (br s, 2H), 0.95 (s, 9H), 0.01 (s, 6H).

MS: Calcd. for $C_{19}H_{29}FNOSi$: 331.1768. Found: 331.1776.

Anal.: Calcd. for $C_{19}H_{29}FNOSi$: C, 68.88; H, 7.86; N, 4.23%. Found: C, 69.15; H, 8.18; N, 4.14%.

Diethyl (3-(3-t-butyldimethylsilyloxymethylphenyl)-4-fluoronitroanilino)methylene malonate

G. A mixture of 5.4 g 3-(3-t-butyldimethylsilyloxymethylphenyl)-4-fluoroaniline and 3.9 ml diethylethoxymethylenemalonate were heated at 150°C for 15 minutes. The mixture was cooled and the resulting oil was purified by elution on silica gel with 25% ethyl acetate/hexanes to give the product as a clear pale green oil (90% yield). NMR ($CDCl_3$, 250MHz): 11.1 (d, 1H, J=9Hz), 8.5 (d, 1H, J=9Hz), 7.45 (m, 5H), 7.2 (m, 2H), 4.82 (s, 2H), 4.3 (m, 4H), 1.35 (m, 6H), 0.95 (s, 9H), 0.15 (s, 6H).

MS: Calcd. for $C_{27}H_{36}FNO_5Si$: 501.2346. Found: 501.2344.

Anal.: Calcd. for $C_{27}H_{36}FNO_5Si$: C, 64.67; H, 7.19; N, 2.79%. Found: C, 64.47; H, 7.06; N, 2.97%.

Ethyl 7-(3-t-butyldimethylsilyloxymethylphenyl)-6-fluoro-4-hydroxyquinoline 3-carboxylate

H. A mixture of diethyl (3-(3-t-butyldimethylsilyloxymethylphenyl)-4-fluoronitroanilino)methylene malonate (2.4 g) in 18 ml Dowtherm A was heated at 260°C for 2.5 hours. The reaction mixture was cooled and triturated with hexanes yielding the product as a white solid (1.13 g, 52% yield), m.p. 310—312°C with decomposition. NMR (1% DMSO-$d_6$/TFA-d, 250 MHz): 9.4 (m, 1H), 8.38 (m, 2H), 7.83 (m, 2H), 7.65 (m, 2H), 5.55 (s, 2H), 5.0 (s, 1H), 4.68 (q, 2H), 1.55 (t, 3H), 1.01 (s, 9H), 0.4 (s, 6H).

Ethyl 7-(3-t-butyldimethylsilyloxymethylphenyl)-1-ethyl-6-fluoro-1,4-dihydroquinol-4-one 3-carboxylate

I. A mixture of 1.13 g ethyl 7-(3-t-butyldimethylsilyloxymethylphenyl)-6-fluoro-4-hydroxyquinoline 3-carboxylate, 3 ml iodoethane and 1.5 g potassium carbonate in 50 ml N,N-dimethylformamide was stirred at room temperature for 24 hours. The reaction mixture was partitioned between ethyl acetate and water. The ethyl acetate layer was washed four times with water and dried. Evaporation gave the crude product which was eluted on silica gel with ethyl acetate/hexanes to yield the pure product as a pale yellow oil (1.02 g, 85% yield). NMR ($CDCl_3$, 250MHz): 8.55 (s, 1H), 8.3 (d, 1H, J=11Hz), 7.5 (m, 5H), 4.85 (s, 2H), 4.45 (q, 2H, J=6.5Hz), 4.3 (q, 3H, J=6.5Hz), 1.6 (t, 3H, J=6.5Hz), 1.45 (t, 3H, J=6.5Hz), 1.0 (s, 9H), 0.0 (s, 6H).

Ethyl 1-ethyl-6-fluoro-7-(3-hydroxymethylphenyl)-1,4-dihydroquinol-4-one 3-carboxylate

J. A 1M solution (2.11 ml) of tetra-n-butylammonium fluoride in tetrahydrofuran was added to a solution of 1.02 g ethyl 7-(3-t-butyldimethylsilyloxymethylphenyl)-1-ethyl-6-fluoro-1,4-dihydroquinol-4-one 3-carboxylate in 20 ml tetrahydrofuran. After 15 minutes, the solution was partitioned between ethyl acetate and water. The organic extracts were dried and evaporated to give the product as a white solid (755 mg, 97% yield).

1-Ethyl-6-fluoro-7-(3-hydroxymethylphenyl)-1,4-dihydroquinol-4-one 3-carboxylic acid

K. A mixture of 755 mg ethyl 1-ethyl-6-fluoro-7-(3-hydroxymethylphenyl)-1,4-dihydroquinol-4-one 3-carboxylate, 50 ml 1M sodium hydroxide and 5 ml tetrahydrofuran were heated at 90°C for 30 minutes. The solution was cooled and acidified to pH 1 with 6M hydrochloric acid. The resulting precipitate was collected by filtration, washed with water and dried, yielding the product as a white solid of m.p. 203—204°C (587 mg, 84% yield). NMR (DMSO-$d_6$, 250MHz): 9.1 (s, 1H), 8.1 (m, 2H), 7.5 (m, 4H), 4.7 (q, 2H), 4.6 (d, 2H, J=7Hz), 1.45 (t, 3H, J=6.5Hz).

MS: Calcd. for $C_{19}H_{16}FNO_4$: 341.1064. Found: 341.1060.

Anal.: Calcd. for $C_{19}H_{16}FNO_4.0.25H_2O$: C, 65.99; H, 4.78; N, 4.05%. Found: C, 66.09; H, 5.16; N, 3.91%.

Example 20

4-(2-Fluorophenyl)-toluene

A. 6.0 g (57% yield) was prepared as an oil by the method of Example 19A from 10 g 2-bromofluorobenzene, 250 mg bis(diphenylphosphinylethane) nickel dichloride and 89 ml 0.73M 4-toluylmagnesium bromide. The product was used without further purification. NMR ($CDCl_3$, 60MHz): 7.5—6.9 (m, 8H), 2.3 (s, 3H).

4-(2-Fluorophenyl)-benzoic acid

B. 1.17 g (100% yield) was prepared by the method of Example 19B from 1.0 g 4-(2-fluorophenyl)-toluene and a white solid of m.p. 226—227°C was recovered. NMR (DMSO-$d_6$, MHz): 8.1 (d, 2H), 7.8—7.3 (m, 6H).

4-(2-Fluoro-5-nitrophenyl)-benzoic acid

C. 1.2 g (100% yield) was prepared by the method of Example 19C from 1.0 g 4-(2-fluorophenyl)-benzoic acid. White solid of m.p. >280°C. NMR (DMSO-$d_6$, 250MHz): 8.5—7.6 (m, 7H).

MS: Calcd. for $C_{13}H_8NFO_4$: 261.0438. Found: 261.0457.

4-(2-Fluoro-5-nitrophenyl)-benzyl alcohol

D. 700 mg (61% yield) was prepared by the method of Example 19D from 1.2 g 4-(2-fluoro-5-nitrophenyl)benzoic acid. Pale yellow solid of m.p. 106—108.5°C. NMR ($CDCl_3$, 250MHz): 8.4 (m, 1H), 8.2 (m, 1H), 7.55 (ABq, 4H), 7.3 (m, 1H), 4.8 (d, 2H, J=6Hz).

3-(4-t-Butyldimethylsilyloxymethylphenyl)-4-fluoronitrobenzene

E. 6.0 g (98% yield was prepared by the method of Example 19E from 4.2 g 4-(2-fluoro-5-nitrophenyl)-benzyl alcohol, 2.5 g imidazole and 3.5 g t-butyldimethylsilyl chloride. The oil was used without further purification.

3-(4-t-butyldimethylsilyloxymethylphenyl)-4-fluoroaniline

F. 5.5 g was prepared by the method of Example 19F from 6.0 g 3-(4-t-butyldimethylsilyloxymethylphenyl)-4-fluoronitrobenzene. The formed oil was used without further purification.

Diethyl 3-(4-t-butyldimethylsilyloxymethylphenyl)-4-fluoroanilino)methylenemalonate

G. 5.12 g (60% yield) was prepared by the method of Example 19G from 5.5 g 3-(4-t-butyldimethylsilyloxymethylphenyl)-4-fluoroaniline and 3.5 ml diethyl ethoxymethylenemalonate. A pale yellow-green oil was obtained. NMR ($CDCl_3$, 250MHz): 8.5 (d, 1H, J=12Hz), 7.5 (ABq, 4H), 7.2 (m, 3H), 4.8 (s, 2H), 4.25 (m, 4H), 1.35 (m, 6H), 1.0 (s, 9H), 0.1 (s, 6H).

Ethyl 7-(4-t-butyldimethylsilyloxymethylphenyl)-6-fluoro-4-hydroxyquinoline 3-carboxylate

H. 379 mg (42% yield) was prepared by the method of Example 19H from 1.0 g diethyl (3-(4-t-butyldimethylsilyloxymethylphenyl)-4-fluoroanilino)methylenemalonate. A white solid fo m.p. >280°C was recovered.

Ethyl 7-(4-t-butyldimethylsilyloxymethylphenyl)-1-ethyl-6-fluoro-1,4-dihydroquyinol-4-one 3-carboxylate

I. 233 mg (58% yield) was prepared as a solid of m.p. 158—160°C by the method of Example 19I from 379 mg ethyl 7-(4-t-butyldimethylsilyloxymethylphenyl)-6-fluoro-4-hydroxyquinoline 3-carboxylate. NMR ($CDCl_3$, 250MHz): 8.5 (s, 1H), 8.2 (d, 1H, J=11Hz), 7.5 (ABq, 4H), 7.45 (d, 1H), 4.8 (s, 2H), 4.4 (q, 2H, J=6.5Hz), 4.3 (q, 2H, J=6.5Hz), 1.55 (t, 3H, J=6.5Hz), 1.4 (t, 3H, J=6.5Hz), 1.0 (s, 9H), 0.1 (s, 6H).

Ethyl 1-ethyl-6-fluoro-7-(4-hydroxymethylphenyl)-1,4-dihydroquinol-4-one 3-carboxylate

J. 170 mg (97% yield) was prepared by the method of Example 19J from 230 mg ethyl 7-(4-t-butyldimethylsilyloxymethylphenyl)-1-ethyl-6-fluoro-1,4-dihydroquinol-4-one 3-carboxylate as a white solid which was used without further purification.

1-Ethyl-6-fluoro-7-(4-hydroxymethylphenyl)-1,4-dihydroquinol-4-one 3-carboxylic acid

K. 155 mg (95% yield) was prepared by the method of Example 19K from 170 mg ethyl 1-ethyl-6-fluoro-7-(4-hydroxymethylphenyl)-1,4-dihydroquinol-4-one 3-carboxylate. A white solid of m.p. 220—221°C was recovered after recrystallisation from dimethyl formamide. NMR (DMSO-$d_6$, 250MHz): 9.1 (s, 1H), 8.1 (dd, 2H), 8.6 (ABq, 4H), 4.7 (q, 2H), 4.6 (s, 2H), 1.45 (t, 3H, J=6.5Hz).

## Example 21

Ethyl 1-ethyl-6-fluoro-7-(4-formylphenyl)-1,4-dihydroquinol-4-one 3-carboxylate

A. Oxalyl chloride (171 mg) was added dropwise to a solution of ethyl 1-ethyl-6-fluoro-7-(4-hydroxymethylphenyl)-1,4-dihydroquinol-4-one 3-carboxylate (450 mg) in 8 ml dichloromethane and 2 ml dimethyl sulfoxide at −78°C. After 25 minutes, 0.9 ml triethylamine was added, the mixture warmed to room temperature and partitioned between water and ethyl acetate. The organic phase was dried and concentrated to a small volume. The product was precipitated by addition of diethyl ether as a white solid of m.p. 165—170°C (360 mg, 80% yield). NMR ($CDCl_3$, 250MHz): 10.1 (s, 1H), 8.55 (s, 1H), 8.3 (d, 1H, J=11Hz), 8.0 (d, 2H, J=9Hz), 7.75 (d, 2H, J=9Hz), 7.5 (d, 1H, J=6Hz), 4.4 (2q, 4H), 1.6 (t, 3H, J=6.5Hz), 1.45 (t, 3H, J=6.5Hz).

MS: Calcd. for $C_{21}H_{18}NO_4F$: 367.1220. Found: 367.1230.

1-Ethyl-6-fluoro-7(4-formylphenyl)-1,4-dihydroquinol-4-one 3-carboxylic acid

B. 63 mg (62% yield) was prepared by the method of Example 19K from 110 mg ethyl 1-ethyl-6-fluoro-7-(4-formylphenyl)-1,4-dihydroquinol-4-one 3-carboxylate as a white solid of m.p. >270°C.

NMR (DMSO-$d_6$/Trifluoroacetic acid-d, 250MHz): 9.45 (s, 1H), 8.5 (d, 1H, J=11Hz), 8.4 (d, 1H, J=6Hz), 8.2 (d, 2H, J=9Hz), 8.0 (d, 2H, J=9Hz), 5.0 (m, 2H), 1.8 (t, 3H, J=6.5Hz).

MS: Calcd. for $C_{19}H_{14}FNO_4$: 339.0908. Found: 339.0872.

Anal.: Calcd. for $C_{19}H_{14}FNO_4$: C, 67.26; H, 4.13; N, 4.13%. Found: C, 67.08; H, 4.45; N, 4.04%.

## Example 22

4-Bromo-2-chloroanisole

A. A solution of 4-bromo-2-chlorophenol (5.18 g) in 20 ml tetrahydrofuran was added dropwise to a stirred mixture of sodium hydride (0.72 g) in tetrahydrofuran (50 ml). After 30 minutes, iodomethane (4.26 g) was added and the mixture was stirred overnight at room temperature. A further 7.1 g of iodomethane

was added and the mixture was heated at reflux overnight. The reaction mixture was then partitioned between water and chloroform. The combined organic extracts were dried and evaporated to yield the product after washing with hexanes as a white solid (5.4 g, 98% yield) of m.p. 63°C. Anal.: Calcd. for $C_7H_6BrC_{10}$: C, 38.00; H, 2.71%. Found: C, 38.07; H, 2.80%.

Ethyl 1-ethyl-7-(3-chloro-4-methoxyphenyl)-6,8-difluoro-1,4-dihydroquinol-4-one 3-carboxylate

B. 300 mg (35.5% yield) was prepared by the method of Example 2 using 1.11 g 4-bromo-2-chloroanisole, 5 ml 2M t-butyl lithium solution, 750 mg zinc chloride and 720 mg ethyl 7-bromo-6,8-difluoro-1-ethyl-1,4-dihydroquinol-4-one 3-carboxylate and 100 mg tetrakis(triphenylphosphine)palladium. A yellow solid of m.p. 280°C was recovered.

NMR ($CDCl_3$, 250MHz): 8.45 (s, 1H), 8.15 (dd, 1H, J=2Hz and 9Hz), 7.55 (s, 1H), 7.4 (m, 1H), 7.1 (d, 1H, J=9Hz), 4.4 (2q, 4H), 4.0 (s, 3H), 1.55 (t, 3H, J=6.5Hz), 1.45 (t, 3H, J=6.5Hz).

Anal.: Calcd. for $C_{21}H_{18}ClF_2NO_4.0.5H_2O$: C, 58.00; H, 4.41; N, 3.25%. Found: C, 59.06; H, 4.52; N, 3.17%.

7-(3-chloro-4-methoxyphenyl)-6,8-difluoro-1-ethyl-1,4-dihydroquinol-4-one 3-carboxylic acid

C. 250 mg (90% yield) was prepared by the method of Example 13B from 300 mg of ethyl 1-ethyl-7-(3-chloro-4-methoxyphenyl)-6,8-difluoro-1,4-dihydroquinol-4-one 3-carboxylate. A white solid was recovered having a m.p. >280°C.

Anal.: Calcd. for $C_{19}H_{14}ClF_2NO_4.1/3H_2O$: C, 57.14; H, 3.67; N, 3.50%. Found: C, 57.16; H, 3.69; N, 3.22%. NMR (DMSO-$d_6$, 250MHz): 9.05 (s, 1H), 8.05 (dd, 1H, 9Hz and 2Hz), 7.7 (s, 1H), 7.55 (m, 1H), 7.35 (d, 1H, J=9Hz), 4.65 (m, 2H), 3.95 (s, 3H), 1.45 (t, 3H, J=6.5Hz).

## Example 23

7-(3-chloro-4-hydroxyphenyl)-6,8-difluoro-1-ethyl-1,4-dihydroquinol-4-one 3-carboxylic acid

40 mg (21% yield) was prepared by the method of Example 14 from 200 mg of 7-(3-chloro-4-methoxyphenyl)-6,8-difluoro-1-ethyl-1,4-dihydroquinol-4-one 3-carboxylic acid. Yellow solid of m.p. 235—238°C.

NMR (DMSO-$d_6$, 250MHz): 9.05 (s, 1H), 8.05 (dd, 1H, J=2Hz and 9Hz), 7.65 (s, 1H), 7.4 (d, 1H, J=9Hz), 7.15 (d, 1H, J=9Hz), 4.65 (m, 2H), 1.45 (t, 3H, J=6.5Hz).

MS: Calcd. for $C_{18}H_{12}ClF_2NO_4$: 379.0423. Found: 379.0423.

## Example 24

Ethyl 6,8-difluoro-1-ethyl-7-(2-methoxyphenyl)-1,4-dihydroquinol-4-one 3-carboxylate

A. 504 Mg (65% yield) was prepared by the method of Example 2 using 935 mg of 2-bromoanisole, 5 ml 2M t-butyl lithium, 818 mg anhydrous zinc chloride, 720 mg ethyl 7-bromo-6,8-difluoro-1,4-dihydroquinol-4-one 3-carboxylate and 248 mg tetrakis(triphenylphosphine)-palladium. NMR ($CDCl_3$, 250MHz): 8.4 (s, 1H), 8.0 (dd, 1H, J=2Hz and 9Hz), 7.2 (m, 4H), 4.4 (2q, 4H), 3.8 (s, 3H), 1.5 (t, 3H, J=6Hz), 1.4 (t, 3H, J=6Hz).

MS: Calcd. for $C_{21}H_{19}F_2NO_4$: 387.1285. Found: 387.1306.

6,8-Difluoro-1-ethyl-7-(2-methoxyphenyl)-1,4-dihydroquinol-4-one 3-carboxylic acid

B. 343 Mg (74% yield) was prepared by the method of Example 1F from 500 mg ethyl 6,8-difluoro-1-ethyl-7-(2-methoxyphenyl)-1,4-dihydroquinol-4-one 3-carboxylate as a white solid of m.p. >260°C. Anal.: Calcd. for $C_{19}H_{15}F_2NO_4.1H_2O$: C, 60.47; H, 4.50; N, 3.71%. Found: C, 60.73; H, 4.17; N, 3.82%.

NMR (DMSO-$d_6$, 250MHz): 9.05 (s, 1H), 8.05 (dd, 1H, J=2Hz and 9Hz), 7.55 (m, 1H), 7.45 (d, 1H, J=9Hz), 7.25 (d, 1H), J=9Hz), 7.15 (m, 1H), 4.65 (m, 2H), 3.8 (s, 3H, 1.45 (t, 3H, J=6.5Hz).

MS: Calcd. for $C_{19}H_{15}F_2NO_4$: 359.0969. Found: 359.0963.

## Example 25

6,8-Difluoro-1-ethyl-7-(2-hydroxyphenyl)-1,4-dihydroquinol-4-one 3-carboxylic acid

135 Mg (45% yield) was prepared by the method of Example 12 from 310 mg 6,8-difluoro-1-ethyl-7-(2-methoxyphenyl)-1,4-dihydroquinol-4-one 3-carboxylic acid. A cream solid of m.p. >260°C was recovered. Anal.: Calcd. for $C_{18}H_{13}F_2NO_4.1/4H_2O$: C, 61.80; H, 3.71; N, 4.00%. Found: C, 61.54; H, 3.98; N, 3.91%.

NMR (DMSO-$d_6$, 250MHz): 10 (br s, 1H), 9.1 (s, 1H), 8.05 (dd, 1H, J=2Hz and 9Hz), 7.35 (m, 2H), 7.0 (m, 2H), 4.65 (m, 2H), 1.45 (t, 3H, J=6Hz).

MS: Calcd. for $C_{18}H_{13}F_2NO_4$: 345.0911. Found: 345.0911.

## Example 26

3-Bromo-4-fluoroaniline

A. 10.68 G (95% yield) was prepared by the method of Example 1B from 13 g of 3-bromo-4-fluoronitrobenzene. The product was an oil which was used without further purification. NMR ($CDCl_3$, 250MHz): 6.9 (m, 2H), 6.6 (m, 1H).

Ethyl 7-bromo-6-fluoro-4-hydroxyquinoline 3-carboxylate

B. 8.8 G (55% yield) was prepared by the method of Example 1C from 10 g 3-bromo-4-fluoroaniline and

11.36 g diethyl ethoxymethylene. A white solid of m.p. >280°C was recovered. NMR (DMSO-$d_6$/Trifluoroacetic acid-d, 250MHz): 9.35 (s, 1H), 8.6 (d, 1H, J=6Hz), 8.3 (d, 1H, J=11Hz), 4.65 (q, 2H, J=6.5Hz), 1.5 (t, 3H, J=6.5Hz).

MS: Calcd. for $C_{12}H_9FBrNO_3$: 312.9759. Found: 312.9766.

Ethyl 7-bromo-1-ethyl-6-fluoro-1,4-dihydroquinol-4-one 3-carboxylate

C. 5.2 G (55% yield) was prepared by the method of Example 1D from 8.6 g ethyl 7-bromo-6-fluoro-4-hydroxyquinoline 3-carboxylate. White solid of m.p. 149—150°C.

NMR ($CDCl_3$, 250MHz): 8.45 (s, 1H), 8.15 (d, 1H, J=11Hz), 7.7 (d, 1H, J=6Hz), 4.4 (q, 2H, J=6.5Hz), 4.2 (q, 2H, J=6.5Hz), 1.55 (t, 3H, J=6.5Hz), 1.4 (t, 3H, J=6.5Hz).

MS: Calcd. for $C_{14}H_{13}BrFNO_3$: 341.0062. Found: 341.0023.

Ethyl 1-ethyl-6-fluoro-7-(4-methoxyphenyl)-1,4-dihydroquinol-4-one 3-carboxylate

D. 422 mg (64% yield) was prepared by the method of Example 13A from 500 mg 4-bromoanisole, 2.67 ml 2M t-butyl lithium, 500 mg anhydrous zinc chloride, 608 mg ethyl 7-bromo-1-ethyl-6-fluoro-1,4-dihydroquinol-4-one 3-carboxylate, and 300 mg tetrakis (triphenylphosphine)palladium. Yellow solid of m.p. 215—218°C. NMR ($CDCl_3$, 250MHz): 8.45 (s, 1H), 8.2 (d, 1H, J=11Hz), 7.55 (dd, 2H, J=9Hz and 2Hz), 7.45 (d, 1H, J=6Hz), 7.0 (d, 2H, J=9Hz), 4.4 (q, 2H, J=6.5Hz), 4.3 (q, 2H, J=6.5Hz), 3.9 (s, 3H), 1.6 (t, 3H, J=6.5Hz), 1.45 (t, 3H, J=6.5Hz).

1-Ethyl-6-fluoro-7-(4-methoxyphenyl)-1,4-dihydroquinol-4-one 3-carboxylic acid ($R_1$=$R_2$=H; Y=ethyl; $R_3$=4-methoxyphenyl)

E. 355 mg (96% yield) was prepared by the method of Example 13B from 400 mg ethyl 1-ethyl-6-fluoro-7-(4-methoxyphenyl)-1,4-dihydroquinol-4- one 3-carboxylate. White solid of m.p. 279—282°C.

NMR (DMSO-$d_6$/Trifluoroacetic acid-d, 250MHz): 9.4 (s, 1H), 8.4 (d, 1H, J=11Hz), 8.3 (d, 1H, J=6Hz), 7.75 (d, 2H, J=9Hz), 7.2 (d, 2H, J=9Hz), 5.0 (m, 2H), 4.0 (s, 3H), 1.8 (t, 3H, J=6.5Hz).

MS: Calcd. for $C_{19}H_{16}FNO_4$: 341.1064. Found: 341.1057.

Anal.: Calcd. for $C_{19}H_{16}FNO_4$: C, 65.14; H, 4.86; N, 4.00%. Found: C, 65.19; H, 4.74; N, 3.91%.

## Example 27

1-Ethyl-6-fluoro-7-(4-hydroxyphenyl)-1,4-dihydroquinol-4-one 3-carboxylic acid

276 Mg (96% yield) was prepared by the method of Example 14 from 300 mg 1-ethyl-6-fluoro-7-(4-methoxyphenyl)-1,4-dihydroquinol-4-one 3-carboxylic acid. Yellow solid of m.p. >280°C. NMR (DMSO-$d_6$/trifluoroacetic acid-d, 250MHz): 9.0 (s, 1H), 8.0 (d, 1H, J=11Hz), 7.85 (d, 1H, J=6Hz), 7.3 (d, 2H, J=9Hz), 6.75 (d, 2H, J=9Hz), 4.55 (m, 2H), 1.4 (t, 3H, J=6Hz).

MS: Calcd. for $C_{18}H_{14}FNO_4$: 327.0907. Found: 327.0930. Anal.: Calcd. for $C_{18}H_{14}FNO_4 \cdot 1/8H_2O$: C, 65.60; H, 4.32; N, 4.25%. Found: C, 65.65; H, 4.43; N, 4.10%.

## Example 28

Ethyl 1-ethyl-6-fluoro-7-phenyl-1,4-dihydroquinol-4-one 3-carboxylate

173 mg (51% yield) was prepared by the method of Example 1E from 0.72 ml 1.83 M phenyl lithium, 300 mg ethyl 7-bromo-6-fluoro-1,4-dihydroquinol-4-one 3-carboxylate, 192 mg zinc chloride and 104 mg tetrakis-(triphenylphosphine) palladium. This sample was identical in every respect with that prepared in Example 4E.

MS: Calcd. for $C_{20}H_{18}FNO_3$: 339.1271. Found: 339.1257.

## Example 29

Ethyl 7-bromo-6,8-difluoro-1-(2-fluoroethyl)-1,4-dihydroquinol-4-one 3-carboxylate

A. By the method of Example 1D, ethyl 7-bromo-6,8-difluoro-4-hydroxyquinoline-3-carboxylate (5.0 g, 15 mmol), anhydrous potassium carbonate (4.2 g, 30 mmol), 1-fluoro-2-bromo-ethane (25 g, 15 ml, 200 mmol) in dimethylformamide (50 ml) were reacted to give a white solid after recrystallization from 2-propanol (3.3 g, 58% yield), m.p. 185—187°C; IR ($CHCl_3$, cm-1): 1732 (s), 1695 (s), 1461 (s); NMR ($CDCl_3$, 250MHz): 8.39 (s, 1H), 8.14 (dd, 1H, J=10, 4Hz), 4.95—4.85 (m, 2H), 4.78—4.55 (m, 2H), 4.40 (q, 2H, J=7Hz), 1.40 (t, 3H, J=7Hz).

Anal.: Calcd. for $C_{14}H_{11}BrF_3NO_3$: C, 44.49, H, 2.93, N, 3.71, Br, 21.14, F, 15.08. Found: C, 44.35, H, 2.98, N, 3.64, Br, 21.50, F, 14.65.

Ethyl 6,8-difluoro-7-(4-methoxyphenyl)-1-(2-fluoroethyl)-1,4-dihydroquinol-4-one 3-carboxylate

B. By the method of Example 2, ethyl 7-bromo-6,8-difluoro-1-(2-fluoroethyl)-1,4-dihydroquinol-4-one 3-carboxylate (1.0 g, 2.7 mmol), 1-bromo-4-methoxybenzene (1.24 g, 0.83 ml, 6.6 mmol), a solution of t-butyl lithium in n-pentane (1.8 M, 7.4 ml, 13.3 mmol), anhydrous zinc chloride (1.1 g, 8.8 mmol) and tetrakis (triphenylphosphine)palladium (306 mg) were reacted to give crude product as a yellow solid. Chromatography on silica gel with ethyl acetate afforded a solid (507 mg, 47% yield); m.p. 193—195°C; IR (KBr, cm-1): 1726 (s), 1612 (s), 1575 (m), 1541 (m), 1518 (m); NMR ($CDCl_3$, 250MHz): 8.42 (s, 1H, 8.16 (dd, 1H,

J=10, 4Hz), 7.43 (d, 2H, J=9Hz), 7.05 (d, 2H, J=9Hz), 4.95—4.65 (m, 4H), 4.42 (q, 2H, J=7Hz), 3.91 (s, 3H), 1.42 (t, 3H, J=7Hz).

Anal.: Calcd. for $C_{21}H_{18}F_3NO_4.H_{20}$: C, 59.57, H, 4.76, N, 3.31. Found: C, 59.83, H, 4.33, N, 3.28.

6,8-Difluoro-7-(4-methoxyphenyl)-1-(2-fluoroethyl)-1,4-dihydroquinol-4-one 3-carboxylic acid

C. By the method of Example 13B, ethyl 6,8-difluoro-7-(4-methoxyphenyl)-1-(2-fluoroethyl)-1,4-dihydroquinol-4-one 3-carboxylate (275 mg, 0.7 mmol) was hydrolyzed to give a white solid (191 mg, 75% yield); m.p. >270°C; IR (KBr, cm-1): 1720 (s), 1621 (s), 1612 (s); NMR (1% DMSO-$d_6$/$CF_3CO_2D$, 250MHz): 8.90 (s, 1H), 7.90 (d, 1H, J=9Hz), 7.22 (d, 2H, J=10Hz), 6.80 (d, 2H, J=10Hz), 4.95 (br. d, 2H, J=24Hz), 4.65 (d, 2H, J=45Hz), 3.57 (s, 3H).

Anal.: Calcd. for $C_{19}H_{14}F_3NO_4.0.75H_2O$: C, 58.39, H, 3.99, N, 3.58. Found: C, 58.12, H, 3.75, N, 3.38.

Example 30

6,8-Difluoro-7-(4-hydroxyphenyl)-1-(2-fluoroethyl)-1,4-dihydroquinol-4-one 3-carboxylic acid

By the method of Example 14, 6,8-difluoro-7-(4-methoxyphenyl)-1-(2-fluoroethyl)-1,4-dihydroquinol-4-one 3-carboxylic acid (200 mg, 0.53 mmol) and a solution of boron tribromide in methylene chloride (1M, 5.3 ml, 5.3 mmol) were reacted to give crude product as a yellow solid. Trituration with boiling water afforded a white solid (131 mg, 66% yield); m.p. >280°C; IR (KBr, cm-1): 1724 (s), 1610 (s), 1589 (s), 1567 (m); NMR (1% DMSO-$d_6$/$CF_3CO_2D$, 250MHz): 9.35 (s, 1H), 8.34 (d, 1H, J=10Hz), 7.55 (d, 2H, J=9Hz), 7.20 (d, 2H, J=9Hz), 5.38 (d, 2H, J=24Hz), 5.00 (d, 2H, J=45Hz); MS: calcd. for $C_{18}H_{12}F_3NO_4$: 363.0719. Found: 363.0709.

Anal.: Calcd. for $C_{18}H_{12}F_3NO_4.H_{20}$: C, 56.70, H, 3.70, N, 3.67. Found: C, 56.99, H, 3.45, N, 3.67.

Example 31

Ethyl 7-bromo-6,8-difluoro-1-methyl-1,4-dihydroquinol-4-one 3-carboxylate

A. By the method of Example 1D, ethyl 7-bromo-6,8-difluoro-4-hydroxyquinolinone-3-carboxylate (5.0 g, 15 mmol), anhydrous potassium carbonate (4.16 g, 30 mmol) and iodomethane (6.4 g, 2.8 ml, 45 mmol) in dimethylformamide (50 ml) were reacted to give a white solid, after recrystallization from 2-propanol (3.4 g, 66% yield); m.p. 174—175°C; IR ($CHCl_3$, cm-1); 1732 (s), 1693 (s), 1642 (s), 1611 (s); NMR ($CDCl_3$, 250MHz): 8.40 (s, 1H), 8.00 (dd, 1H, J=10, 4Hz), 4.35 (q, 2H, J=7Hz), 4.08 (d, 3H, J=8Hz), 1.58 (t, 3H, J=7Hz).

Anal.: Calcd. for $C_{13}H_{10}BrF_2NO_3$: C, 45.13, H, 2.92, N, 4.05, Br, 23.10, F, 10.98. Found: C, 45.05, H, 2.70, N, 4.01, Br, 22.83, F; 10.52.

Ethyl 6,8-difluoro-7-(4-methoxyphenyl)-1-methyl-1,4-dihydroquinol-4-one 3-carboxylate

B. By the method of Example 2, ethyl 7-bromo-6,8-difluoro-1-methyl-1,4-dihydroquinol-4-one-3-carboxylate (1.0 g, 2.9 mmol), 1-bromo-4-methoxybenzene (1.35 g, 0.91 ml, 7.3 mmol), a solution of t-butyl lithium in pentane (1.8 M, 8.1 ml, 14.6 mmol) and anhydrous zinc chloride (1.31 g, 9.6 mmol) and tetrakis (triphenylphosphine)palladium (306 mg) were reacted to yield crude product as a yellow solid. Column chromatography with ethyl acetate on silica gel afforded a solid (624 mg, 57% yield); m.p. 176—178°C; IR (KBr, cm-1): 1733 (m), 1684 (s), 1639 (s), 1617 (s), 1540 (m); NMR ($CDCl_3$, 250MHz): 8.38 (s, 1H), 8.08 (dd, 1H, J=10, 4Hz), 7.42 (d, 2H, J=8Hz), 7.05 (d, 2H, J=8Hz), 4.41 (q, 2H, J=7Hz), 4.12 (d, 3H, J=8Hz), 3.90 (s, 3H), 1.42 (t, 3H, J=7Hz); MS (m/e): 373 (parent), 201 (base).

Anal.: Calcd. for $C_{20}H_{13}F_2NO_4.0.5H_2O$: C, 62.83, H, 4.74, N, 3.66. Found: C, 63.22, H, 4.77, N, 3.42.

6,8-Difluoro-7-(4-methoxyphenyl)-1-methyl-1,4-dihydroquinol-4-one 3-carboxylic acid

C. By the method of Example 13B, ethyl 6,8-difluoro-7-(4-methoxyphenyl)-1,4-dihydroquinol-4-one 3-carboxylate (446 mg, 1.2 mmol), was hydrolyzed to give a white solid (332 mg, 80% yield); m.p. >280°C; IR (KBr, cm-1): 1718 (s), 1612 (s), 1567 (s), 1539 (s); NMR ($CF_3CO_2D$, 250MHz): 9.30 (s, 1H), 8.25 (d, 1H, J=10Hz), 7.65 (d, 2H, J=10Hz), 7.22 (d, 2H, J=10Hz), 4.70 (d, 3H, J=10Hz), 3.96 (s, 3H); MS: Calcd. for $C_{18}H_{13}F_2NO_4$: 345.0812. Found: 345.0821.

Anal.: Calcd. for $C_{18}H_{13}F_2NO_4$: 62.61, H, 3.79, N, 4.06. Found: C, 62.43, H, 3.81, N, 3.95.

Example 32

6,8-Difluoro-7-(4-hydroxyphenyl)-1-methyl-1,4-dihydroquinol-4-one 3-carboxylic acid

By the method of Example 14, 6,8-difluoro-7-(4-methoxyphenyl)-1-methyl-1,4-dihydroquinol-4-one 3-carboxylic acid (202 mg, 0.6 mmol) and a solution of boron tribromide in methylene chloride (1M, 5.9 ml, 5.9 mmol) were reacted to give crude product as a yellow solid. Trituration with hot water afforded a white solid (126 mg, 65% yield); m.p. >270°C; NMR ($CF_3CO_2D$, 250MHz): 9.34 (s, 1H), 8.30 (d, 1H, J=10Hz), 7.58 (d, 2H, J=10Hz), 7.20 (d, 2H, J=10Hz), 4.73 (d, 3H, J=10Hz); MS: calcd. for $C_{16}H_{11}F_2NO_2$ (parent—$CO_2$): 287.0758. Found: 287.0751.

Anal.: Calcd. for $C_{17}H_{11}F_2NO_4.0.25H_2O$: C, 60.81, H, 3.45, N, 4.17. Found: C, 60.46, H, 3.33, N, 4.05.

Example 33

Ethyl 7-bromo-6,8-difluoro-1-propyl-1,4-dihydroquinol-4-one 3-carboxylate

A. By the method of Example 1D, ethyl 7-bromo-6,8-difluoro-4-hydroxyquinoline 3-carboxylate (5.0 g,

15 mmol), anhydrous potassium carbonate (4.16 g, 30 mmol) and 1-bromopropane (11 g, 8.2 ml, 90 mmol) in dimethylformamide (50 ml) were reacted to give a white solid, after recrystallization from 2-propanol (2.7 g, 48% yield); m.p. 139—140°C; IR ($CHC_{13}$, cm-1): 1731 (s), 1692 (s), 1641 (s), 1610 (s); NMR ($CDCl_3$, 250MHz): 8.40 (s, 1H), 8.15 (dd, 1H, J=10, 4Hz), 4.42 (q, 2H, J=7Hz), 4.35—4.25 (m, 2H), 1.94 (sextet, 2H, J=7Hz), 1.42 (t, 3H, J=7Hz).

Anal.: Calcd. for $C_{15}H_{14}BrF_2NO_3$: C, 48.17, H, 3.77, N, 3.75, Br, 21.37, F, 10.16. Found: C, 47.86, H, 3.73, N, 3.84, Br, 21.10, F, 10.35.

Ethyl 6,8-difluoro-7-(4-methoxyphenyl)-1-propyl-1,4-dihydroquinol-4-one 3-carboxylate

B. By the method of Example 2, ethyl 7-bromo-6,8-difluoro-1-propyl-1,4-dihydroquinol-4-one 3-carboxylate (1.0 g, 2.7 mmol), 1-bromo-4-methoxybenzene (1.26 g, 0.84 ml, 6.75 mmol), anhydrous zinc chloride (1.1 g, 8.1 mmol), a solution of t-butyl lithium in pentane (1.8 M, 7.5 ml, 13.5 mmol) and tetrakis(triphenylphosphine) palladium (320 mg) were reacted to give crude product as a yellow solid. Chromatography with ethyl acetate on silica gel afforded a pale yellow solid (360 mg, 73% yield); m.p. 126—128°C; IR (KBR, cm-1): 1734 (s), 1612 (s), 1574 (m); NMR ($CDCl_3$, 250MHz): 8.45 (s, 1H), 8.15 (dd, 1H, J=10, 3Hz), 7.42 (d, 2H, J=8Hz), 7.05 (d, 2H, J=8Hz), 4.44 (q, 2H, J=7Hz), 3.92 (s, 3H), 3.35—3.25 (m, 2H), 2.00—1.85 (m, 2H), 1.45 (t, 3H, J=7Hz), 1.00 (t, 3H, J=7Hz).

| | | | |
|---|---|---|---|
| Anal.: Calcd. for $C_{22}H_{21}F_2NO_4 \cdot 0.25H_2O$: | C, 65.10; | H, 5.34, | N, 3.45. |
| Found: | C, 65.22, | H, 5.24, | N, 3.38. |

6,8-Difluoro-7-(4-methoxyphenyl)-1-propyl-1,4-dihydroquinol-4-one 3-carboxylic acid

C. By the method of Example 13B, ethyl 6,8-difluoro-7-(4-methoxyphenyl)-1-propyl-1,4-dihydroquinol-4-one 3-carboxylate (245 mg, 0.6 mmol) was hydrolyzed to give a white solid (154 mg, 68% yield); m.p. 272—274°C; IR (KBr, cm-1): 1722 (s), 1612 (s); NMR (1% $DMSO-d_6/CF_3CO_2D$, 250MHz): 9.34 (s, 1H), 8.30 (d, 1H, J=8Hz), 7.62 (d, 2H, J=8Hz), 7.22 (d, 2H, J=8Hz), 5.10—4.88 (m, 2H), 3.96 (s, 3H), 2.25—2.10 (m, 2H), 1.12 (t, 3H, J=7Hz). MS: Calcd. for $C_{20}H_{17}F_2NO_4$: 373.1126. Found: 373.1146.

| | | | |
|---|---|---|---|
| Anal.: Calcd. for $C_{20}H_{17}F_2NO_4$: | C, 64.34, | H, 4.59, | N, 3.75. |
| Found: | C, 63.91, | H, 4.49, | N, 3.67. |

## Example 34

6,8-Difluoro-7-(4-hydroxyphenyl)-1-propyl-1,4-dihydroquinol-4-one 3-carboxylic acid

By the method of Example 14, 6,8-difluoro-7-(4-methoxyphenyl)-1-propyl-1,4-dihydroquinol-4-one 3-carboxylic acid (103 mg, 0.28 mmol) and a solution of boron tribromide in methylene chloride (1 M, 2.8 ml, 2.8 mmol) were reacted to give crude product as a yellow solid. Trituration with boiling water and filtration afforded a white solid (98 mg, 98% yield); m.p. 262—264°C; IR (KBr, cm-1): 1721 (s), 1614 (m), 1561 (m); NMR (1% $DMSO-d_6/CF_3CO_2D$, 250MHz): 9.35 (s, 1H), 8.30 (d, 1H, J=8Hz), 7.62 (d, 2H, J=8Hz), 7.20 (d, 2H, J=8Hz), 5.10—4.95 (m, 2H), 3.98 (s, 3H), 2.20—2.10 (m, 2H), 1.60—1.40 (m, 2H), 1.05 (t, 3H, J=7Hz).

MS: Calcd. for $C_{19}H_{15}F_2NO_4$: 359.0980. Found: 359.0969.

## Example 35

Ethyl 7-bromo-6,8-difluoro-1-butyl-1,4-dihydroquinol-4-one 3-carboxylate

A. By the method of Example 1D, ethyl 7-bromo-6,8-difluoro-4-hydroxyquinoline 3-carboxylate (5.0 g, 15 mmol), anhydrous potassium carbonate (4.2 g, 30 mmol) and 1-bromobutane (18.5 g, 14.4 ml, 135 mmol) in dimethylformamide were reacted to give a white solid, after recrystallization from 2-propanol (3.1 g, 53% yield); m.p. 140—141°C; IR ($CHCl_3$, cm-1): 1731 (s), 1692 (s), 1641 (s), 1610 (s), 1547 (m), 1480 (s); NMR ($CDCl_3$, 250MHz): 8.40 (s, 1H), 8.15 (dd, 1H, J=10, 4Hz), 4.42 (q, 2H, J=7Hz). 4.40—4.25 (m, 2H), 1.95—1.85 (m, 2H), 1.65—1.40 (m, 2H), 1.41 (t, 3H, J=7Hz), 1.00 (t, 3H, J=7Hz).

| | | | | | |
|---|---|---|---|---|---|
| Anal.: Calcd. for $C_{16}H_{16}BrF_2NO_3$: | C, 49.53, | H, 4.16, | N, 3.61, | Br, 20.60, | F, 9.79. |
| Found: | C, 49.65, | H, 4.12, | N, 3.56, | Br, 20.59, | F, 10.21. |

Ethyl 6,8-difluoro-7-(4-methoxyphenyl)-1-butyl-1,4-dihydroquinol-4-one 3-carboxylate

B. By the method of Example 2, ethyl 7-bromo-6,8-difluoro-1-butyl-1,4-dihydroquinol-4-one 3-carboxylate (1 g, 2.6 mmol), 1-bromo-4-methoxybenzene (1.22 g, 0.82 ml, 6.6 mmol), anhydrous zinc chloride (1.1 g, 8.1 mmol), a solution of t-butyl lithium in pentane (1.8 M, 7.3 ml, 13.1 mmol) and tetrakis(triphenylphosphine)palladium (300 mg) were reacted to give crude product as a yellow solid, which was used without further purification.

6,8-Difluoro-7-(4-methoxyphenyl)-1-butyl-1,4-dihydroquinol-4-one 3-carboxylic acid

C. By the method of Example 13B, ethyl 6,8-difluoro-7-(4-methoxyphenyl)-1-butyl-1,4-dihydroquinol-4-one 3-carboxylate was hydrolyzed to give a white solid (134 mg, 13% yield); m.p. 248—249°C; IR (KBr, cm-1): 1721 (s), 1614 (m), 1561 (m), 1538 (m); NMR (1% $DMSO-d_6/CF_3CO_2D$, 250MHz): 9.35 (s, 1H), 8.30 (d, 1H, J=8Hz), 7.62 (d, 2H, J=8Hz), 7.20 (d, 2H, J=8Hz), 5.10—4.95 (m, 2H), 3.98 (s, 3H), 2.20—2.10 (m, 2H), 1.60—1.40 (m, 2H), 1.05 (t, 3H, J=7Hz); MS: 387 (parent), 343 (base).

| | | | |
|---|---|---|---|
| Anal.: Calcd. for $C_{21}H_{19}F_2NO_4 \cdot 0.5H_2O$: | C, 63.63, | H, 5.09, | N, 3.53. |
| Found: | C, 63.73, | H, 4.79, | N, 3.46. |

Example 36

6,8-Difluoro-7-(4-hydroxyphenyl)-1-butyl-1,4-dihydroquinol-4-one 3-carboxylic acid

By the method of Example 14, 6,8-difluoro-7-(4-methoxyphenyl)-1-butyl-1,4-dihydroquinol-4-one 3-carboxylic acid (91 mg, 0.24 mmol) and a solution of boron tribromide (1 M, 2.4 ml, 2.4 mmol) were reacted to give crude product as a yellow solid. Trituration with boiling water, filtration and drying in vacuo afforded a white solid (40 mg, 45% yield); NMR (5% DMSO-$d_6$/$CF_3CO_2D$, 250MHz): 9.46 (s, 1H), 8.35 (d, 1H, J=10Hz), 7.56 (d, 2H, J=10Hz), 7.20 (d, 2H, J=10Hz), 5.10—4.95 (m, 1H), 2.25—2.05 (m, 2H), 1.75—1.55 (m, 2H), 1.10 (t, 3H, J=7Hz); MS: calcd. for $C_{20}H_{17}F_2NO_4$: 373.1126. Found: 373.1082.

| | | | |
|---|---|---|---|
| Anal.: Calcd. for $C_{20}H_{17}F_2NO_4 \cdot 0.75H_2O$: | C, 62.09, | H, 4.82, | N, 3.62. |
| Found: | C, 62.15, | H, 4.82, | N, 3.85. |

Example 37

Ethyl 6,8-difluoro-7-(4-chlorosulfonylphenyl)-1-ethyl-1,4-dihydroquinol-4-one 3-carboxylate

A. By the method of Example 10A, ethyl 6,8-difluoro-7-phenyl-1-ethyl-1,4-dihydroquinol-4-one 3-carboxylate (0.55 g, 1.53 mmol) and chlorosulfonic acid (6 ml) were reacted in methylene chloride (10 ml) to give a yellow oil (0.7 g), which was used without further purification.

Ethyl 6,8-difluoro-7-(4-aminosulfonylphenyl)-1-ethyl-1,4-dihydroquinol-4-one 3-carboxylate

B. Ethyl 6,8-difluoro-7-(4-chlorosulfonylphenyl)-1-ethyl-1,4-dihydroquinol-one 3-carboxylate (0.7 g) was dissolved in dry tetrahydrofuran (10 ml) and the resulting solution was cooled to −78°C. Ammonia (5 ml) was condensed into the reaction mixture with stirring. The reaction mixture was warmed to room temperature and stirred for 16 hours. Solvent was removed in vacuo and the crude product was triturated with water, then ether. Drying in vacuo afforded a pale yellow solid (0.6 g, 90% yield); m.p. >250°C; NMR (3% DMSO-$d_6$/$CF_3CO_2D$, 250MHz): 9.38 (s, 1H), 8.40 (d, 1H, J=8Hz), 8.20 (d, 2H, J=8Hz), 7.95—7.85 (m, 2H), 7.72 (d, 2H, J=8Hz), 5.20—5.05 (m, 2H), 4.72 (q, 4H, J=7Hz), 1.80 (t, 3H, J=7Hz), 1.55 (t, 3H, J=7Hz) MS: Calcd. $C_{20}H_{18}F_2N_2O_5S$: 436.0909. Found: 436.0912.

6,8-Difluoro-7-(4-aminosulfonylphenyl)-1-ethyl-1,4-dihydroquinol-4-one 3-carboxylic acid

C. By the method of Example 10C, ethyl 6,8-difluoro-7-(4-aminosulfonylphenyl)-1-ethyl-1,4-dihydroquinol-4-one-3-carboxylate (0.32 g, 0.73 mmol) and a 1N sodium hydroxide solution were reacted in ethanol (4 ml) to give a white solid (0.25 g, 84% yield); m.p. >250°C; NMR (1% DMSO-$d_6$/$CF_3CO_2D$, 250MHz): 9.45 (s, 1H), 8.40 (d, 1H, J=8Hz), 8.24 (d, 2H, J=8Hz), 8.00—7.85 (m, 2H), 7.85 (m, 2H), 5.25—5.00 (m, 2H), 1.82 (t, 3H, J=7Hz).

MS: Calcd. for $C_{18}H_{14}F_2N_2O_5S$: 408.0580. Found: 408.0560.

Example 38

In addition, the following compounds were prepared:

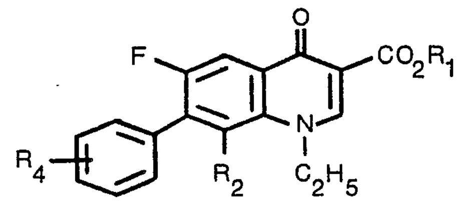

TABLE I

| $R_1$ | $R_2$ | $R_4$ | Melting point in °C. |
|---|---|---|---|
| $C_2H_5$ | F | 3-S(O)$CH_3$ | 165—6 |
| H | F | 3-S(O)$CH_3$ | 277—8 |
| $C_2H_5$ | F | 3-$SO_2CH_3$ | 215—6 |
| H | F | 3-$SO_2CH_3$ | 242—3 |
| H | F | 3,5-di-OH | >260 |
| $C_2H_5$ | H | 3-Cl,4-$OCH_3$ | 227—9 |
| H | H | 3-Cl,4-$OCH_3$ | >260 |
| H | H | 3-Cl,4-OH | >260 |

**Claims for the Contracting States: BE CH/LI DE FR IT LU NL SE GB**

1. A compound selected from those of the formula (I):

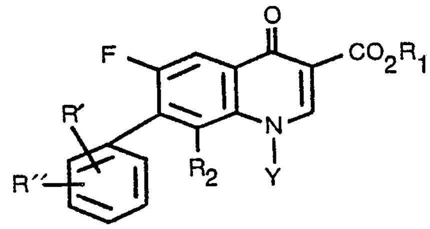

(I)

wherein

$R_1$ is hydrogen, alkyl of 1 to 6 carbon atoms, benzyl or a pharmaceutically acceptable cation;

$R_2$ is hydrogen or fluoro;

Y is selected from alkyl, haloalkyl and polyhaloalkyl of 1 to 3 carbon atoms, hydroxyethyl, cyclopropyl, vinyl, allyl, phenyl, 4-hydroxyphenyl and 4-fluorophenyl;

R′ is hydrogen, alkylsulfinyl of 1 to 4 carbon atoms, alkylsulfonyl of 1 to 4 carbon atoms, hydroxy, alkoxy of 1 to 4 carbon atoms, hydroxyalkyl of 1 to 3 carbon atoms, amino, aminoalkyl of 1 to 3 carbon atoms, formamido, alkanoylamino of 2 or 3 carbon atoms, aminosulfonyl, nitro, formyl, or N-(N′,N′-dimethylformamidino); and

R″ is hydrogen, 3-hydroxy or 3-chloro.

2. A compound according to claim 1 characterized in that said compound is selected from
6,8-difluoro-7-phenyl-1-ethyl-1,4-dihydroquinol-4-one 3-carboxylic acid
6,8-difluoro-7-(4-hydroxyphenyl)-1-ethyl-1,4-dihydroquinol-4-one 3-carboxylic acid
6,8-difluoro-7-(3-chloro-4-hydroxyphenyl)-1-ethyl-1,4-dihydroquinol-4-one 3-carboxylic acid
6,8-difluoro-7-(3-hydroxyphenyl)-1-ethyl-1,4-dihydroquinol-4-one 3-carboxylic acid
6,8-difluoro-7-(4-aminomethylphenyl)-1-ethyl-1,4-dihydroquinol-4-one 3-carboxylic acid
6,8-difluoro-7-(3-aminomethylphenyl)-1-ethyl-1,4-dihydroquinol-4-one 3-carboxylic acid
6,8-difluoro-7-(4-hydroxymethylphenyl)-1-ethyl-1,4-dihydroquinol-4-one 3-carboxylic acid
6,8-difluoro-7-(3-hydroxymethylphenyl)-1-ethyl-1,4-dihydroquinol-4-one 3-carboxylic acid
6,8-difluoro-7-(4-methylsulfinylphenyl)-1-ethyl-1,4-dihydroquinol-4-one 3-carboxylic acid
6,8-difluoro-7-(3-methylsulfonylphenyl)-1-ethyl-1,4-dihydroquinol-4-one 3-carboxylic acid
6,8-difluoro-7-(4-methoxyphenyl)-1-ethyl-1,4-dihydroquinol-4-one 3-carboxylic acid and
6,8-difluoro-7-(3-hydroxymethyl-4-hydroxy)-1-ethyl-1,4-dihydroquinol-4-one 3-carboxylic acid

3. A compound according to claim 1 characterized in that said compound is selected from
6-fluoro-7-phenyl-1-ethyl-1,4-dihydroquinol-4-one 3-carboxylic acid
6-fluoro-7-(4-aminophenyl)-1-ethyl-1,4-dihydroquinol-4-one 3-carboxylic acid
6-fluoro-7-(4-nitrophenyl)-1-ethyl-1,4-dihydroquinol-4-one 3-carboxylic acid
6-fluoro-7-(4-formamidophenyl)-1-ethyl-1,4-dihydroquinol-4-one 3-carboxylic acid
6-fluoro-7-(4-acetamidophenyl)-1-ethyl-1,4-dihydroquinol-4-one 3-carboxylic acid
6-fluoro-7-(4-N-(N′,N′-dimethylformamidino)phenyl)-1-ethyl-1,4-dihydroquinol-4-one 3-carboxylic acid
6-fluoro-7-(4-formylphenyl)-1-ethyl-1,4-dihydroquinol-4-one 3-carboxylic acid
6-fluoro-7-(4-hydroxymethylphenyl)-1-ethyl-1,4-dihydroquinol-4-one 3-carboxylic acid
6-fluoro-7-(3-hydroxymethylphenyl)-1-ethyl-1,4-dihydroquinol-4-one 3-carboxylic acid
6-fluoro-7-(4-aminosulfonylphenyl)-1-ethyl-1,4-dihydroquinol-4-one 3-carboxylic acid.

4. A compound according to claim 1 characterized in that said compound is 6-fluoro-7-(4-hydroxyphenyl)-1-(4-fluorophenyl)-1,4-dihydroquinol-4-one 3-carboxylic acid.

5. An antibacterial composition characterized by comprising a compound according to any one of claims 1 to 4 and a pharmaceutically acceptable carrier.

6. A compound of the formula defined in any one of claims 1 to 4 or a pharmaceutically acceptable addition salt thereof, for use in medicine.

7. Use of a compound of formula defined in any one of claims 1 to 4 or a pharmaceutically acceptable addition salt thereof for manufacture of an antibacterial medicament.

**Claims for the Contracting State: AT**

1. A process for the preparation of 7-substituted arylquinolones of the formula:

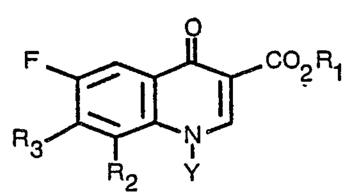

(I),

wherein

$R_1$ is hydrogen, alkyl of 1 to 6 carbon atoms or benzyl or a pharmaceutically acceptable cation;

Y is alkyl, haloalkyl, polyhaloalkyl of 1 to 3 carbon atoms, cyclopropyl, hydroxyethyl, vinyl, allyl,, phenyl, 4-hydroxyphenyl or 4-fluorophenyl;

$R_2$ is hydrogen or fluoro;

$R_3$ is phenyl substituted by R' and R'', and

R' is hydrogen, alkylsulfinyl of 1 to 4 carbon atoms, alkylsulfonyl of 1 to 4 carbon atoms, hydroxy, alkoxy of 1 to 4 carbon atoms, hydroxyalkyl of 1 to 3 carbon atoms, amino, aminoalkyl of 1 to 3 carbon atoms, formamido, alkanoylamino of 2 or 3 carbon atoms, aminosulfonyl, nitro, formyl, or N-(N',N'-dimethylformamidino); and

R'' is hydrogen, 3-hydroxy or 3-chloro.

which comprises

(a) reacting an arylmetallic compound containing aryl group $R_3$ as defined above, except that $R_3$ is not phenyl substituted with alkanoylamino with a compound of the formula:

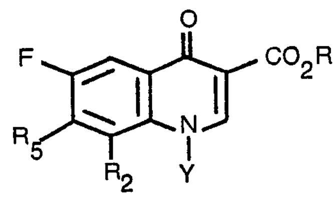

wherein

R is alkyl of 1 to 6 carbon atoms or benzyl;

$R_2$ and Y are as defined above, and $R_5$ is bromo or iodo, in the presence of a transition metal catalyst, or

(b) reacting a compound of the formula:

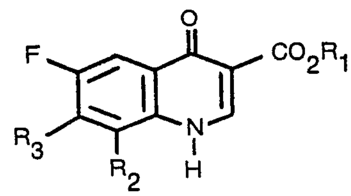

wherein $R_1$, $R_2$ and $R_3$ are as defined above, except that $R_1$ is not hydrogen, with a compound of the formula Y—Hal wherein Y is as defined above and Hal is halogen; and, where necessary,

when $R_3$ is nitrophenyl nitrating a corresponding compound wherein $R_3$ is phenyl;

when $R_3$ is aminophenyl reducing a corresponding compound wherein $R_3$ is nitrophenyl;

when $R_3$ is formamidophenyl reacting a corresponding compound wherein $R_3$ is aminophenyl with formic acid or a formic acid derivative;

when $R_3$ is alkanoylaminophenyl reacting a corresponding compound wherein $R_3$ is aminophenyl with an alkanoyl group containing reagent;

when $R_3$ is aminosulfonylphenyl reacting a corresponding compound wherein $R_3$ is chlorosulfonylphenyl with an amino-introducing reagent, said compound wherein $R_3$ is chlorosulfonylphenyl being obtained by reacting a corresponding compound wherein $R_3$ is phenyl with chlorosulfonic acid;

when $R_3$ is hydroxyphenyl reacting a corresponding compound wherein $R_3$ is methoxyphenyl with an ether-cleaving reagent;

when $R_3$ is hydroxymethylphenyl reacting a corresponding compound wherein $R_3$ is trialkylsilyloxymethylphenyl wherein each alkyl has 1 to 4 carbons with an ether-cleaving reagent;

when $R_3$ is aminomethylphenyl hydrogenating a corresponding compound wherein $R_3$ is azidomethylphenyl;

when $R_3$ is formylphenyl by oxidizing a corresponding compound wherein $R_3$ is hydroxymethylphenyl;

when $R_3$ is alkylsulfinylphenyl or alkylsulfonylphenyl oxidizing a corresponding compound wherein $R_3$ is alkylmercaptophenyl; and

when $R_1$ is hydrogen hydrolyzing a corresponding compound wherein $R_1$ is alkyl or benzyl, or hydrogenolyzing a corresponding compound wherein $R_1$ is benzyl.

2. A process according to claim 1 wherein $R_1$ is hydrogen, Y is 4-fluorophenyl, $R_2$ is hydrogen and $R_3$ is 4-hydroxyphenyl.

3. A process as claimed in claim 1 wherein said reaction (a) is carried out in an ether solvent.

4. A process as claimed in claim 3 wherein said ether solvent is selected from diethylether and tetrahydrofuran.

5. A process as claimed in claim 1 wherein the metal in said arylmetallic compound is magnesium, zinc, cadmium, tin, silver, copper, boron or aluminum.

6. A process as claimed in claim 5 wherein said metal is zinc.

7. A process as claimed in claim 1 wherein said arylmetallic compound is an arylzinc halide.

8. A process as claimed in claim 1, wherein said transition metal catalyst is in the (O) or (II) oxidation state.

9. A process as claimed in claim 8 wherein said transition metal in said catalyst is nickel, palladium or platinum.

10. A process as claimed in claim 8 wherein said transition metal catalyst is a complex containing a transition metal and a phosphorus-containing ligand.

11. A process as claimed in claim 10 wherein said catalyst is tetrakis(triphenylphosphine) palladium.

12. A process as claimed in claim 10 wherein said catalyst is dichlorobis(triphenylphosphine) nickel (II).

13. A compound as formula (I) as defined in claim 1.

**Patentansprüche für die Vertragsstaaten: BH CH/LI DE FR IT LU NL SE GB**

1. Verbindung ausgewählt aus jenen der Formel (I):

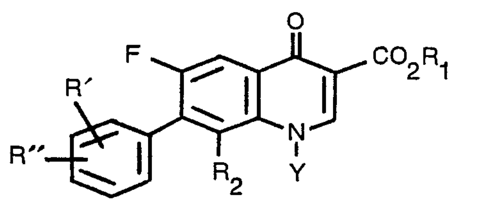

(I),

worin $R_1$ Wasserstoff, Alkyl mit 1 bis 6 Kohlenstoffatomen, Benzyl oder ein pharmazeutisch annehmbares Kation bedeutet, $R_2$ Wasserstoff oder Fluor darstellt, Y ausgewählt ist aus Alkyl, Halogenalkyl und Polyhalogenalkyl mit 1 bis 3 Kohlenstoffatomen, Hydroxyäthyl, Cyclopropyl, Vinyl, Allyl, Phenyl, 4-Hydroxyphenyl und 4-Fluorphenyl, R′ Wasserstoff, Alkylsulfinyl mit 1 bis 4 Kohlenstoffatomen, Alkylsulfonyl mit 1 bis 4 Kohlenstoffatomen, Hydroxy, Alkoxy mit 1 bis 4 Kohlenstoffatomen, Hydroxyalkyl mit 1 bis 3 Kohlenstoffatomen, Amino, Aminoalkyl mit 1 bis 3 Kohlenstoffatomen, Formamido, Alkanoylamino mit 2 oder 3 Kohlenstoffatomen, Aminosulfonyl, Nitro, Formyl oder N-(N′,N′-Dimethylformamidino) ist und R″ Wasserstoff, 3-Hydroxy oder 3-Chlor darstellt.

2. Verbindung nach Anspruch 1, dadurch gekennzeichnet, daß diese Verbindung ausgewählt ist aus:
6,8-Difluor-7-phenyl-1-äthyl-1,4-dihydrochinol-4-on-3-carbonsäure,
6,8-Difluor-7-(4-hydroxyphenyl)-1-äthyl-1,4-dihydrochinol-4-on-3-carbonsäure,
6,8-Difluor-7-(3-chlor-4-hydroxyphenyl)-1-äthyl-1,4-dihydrochinol-4-on-3-carbonsäure,
6,8-Difluor-7-(3-hydroxyphenyl)-1-äthyl-1,4-dihydrochinol-4-on-3-carbonsäure,
6,8-Difluor-7-(4-aminomethylphenyl)-1-äthyl-1,4-dihydrochinol-4-on-3-carbonsäure,
6,8-Difluor-7-(3-aminomethylphenyl)-1-äthyl-1,4-dihydrochinol-4-on-3-carbonsäure,
6,8-Difluor-7-(4-hydroxymethylphenyl)-1-äthyl-1,4-dihydrochinol-4-on-3-carbonsäure,
6,8-Difluor-7-(3-hydroxymethylphenyl)-1-äthyl-1,4-dihydrochinol-4-on-3-carbonsäure,
6,8-Difluor-7-(4-methylsulfinylphenyl)-1-äthyl-1,4-dihydrochinol-4-on-3-carbonsäure,
6,8-Difluor-7-(3-methylsulfonylphenyl)-1-äthyl-1,4-dihydrochinol-4-on-3-carbonsäure,
6,8-Difluor-7-(4-methoxyphenyl)-1-äthyl-1,4-dihydrochinol-4-on-3-carbonsäure und
6,8-Difluor-7-(3-hydroxymethyl-4-hydroxy)-1-äthyl-1,4-dihydrochinol-4-on-3-carbonsäure.

3. Verbindung nach Anspruch 1, dadurch gekennzeichnet, daß diese Verbindung ausgewählt ist aus:
6-Fluor-7-phenyl-1-äthyl-1,4-dihydrochinol-4-on-3-carbonsäure,
6-Fluor-7-(4-aminophenyl)-1-äthyl-1,4-dihydrochinol-4-on-3-carbonsäure,
6-Fluor-7-(4-nitrophenyl)-1-äthyl-1,4-dihydrochinol-4-on-3-carbonsäure,
6-Fluor-7-(4-formamidophenyl)-1-äthyl-1,4-dihydrochinol-4-on-3-carbonsäure,
6-Fluor-7-(4-acetamidophenyl)-1-äthyl-1,4-dihydrochinol-4-on-3-carbonsäure,
6-Fluor-7-{4-[N-(N′,N′-dimethylformamidino)]-phenyl}-1-äthyl-1,4-dihydrochinol-4-on-3-carbonsäure,
6-Fluor-7-(4-formylphenyl)-1-äthyl-1,4-dihydrochinol-4-on-3-carbonsäure,
6-Fluor-7-(4-hydroxymethylphenyl)-1-äthyl-1,4-dihydrochinol-4-on-3-carbonsäure,
6-Fluor-7-(3-hydroxymethylphenyl)-1-äthyl-1,4-dihydrochinol-4-on-3-carbonsäure und
6-Fluor-7-(4-aminosulfonylphenyl)-1-äthyl-1,4-dihydrochinol-4-on-3-carbonsäure.

4. Verbindung nach Anspruch 1, dadurch gekennzeichnet, daß diese Verbindung 6-Fluor-7-(4-hydroxyphenyl)-1-(4-fluorphenyl)-1,4-dihydrochinol-4-on-3-carbonsäure ist.

5. Antibakterielle Zusammensetzung, dadurch gekennzeichnet, daß sie eine Verbindung nach einem der Ansprüche 1 bis 4 und einen pharmazeutisch annehmbaren Träger umfaßt.

6. Verbindung der Formel, wie in einem der Ansprüche 1 bis 4 definiert, oder ein pharmazeutisch annehmbares Additionssalz hievon zur Verwendung in der Medizin.

7. Verwendung einer Verbindung der in einem der Ansprüche 1 bis 4 definierten Formel oder eines pharmazeutisch annehmbaren Additionssalzes hievon zur Herstellung eines antibakteriellen Medikaments.

**Patentansprüche für den Vertragsstaat: AT**

1. Verfahren zur Herstellung von 7-substituierten Arylchinolonen der Formel

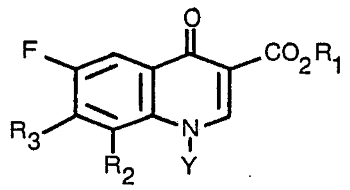

(I),

worin $R_1$ Wasserstoff, Alkyl mit 1 bis 6 Kohlenstoffatomen, Benzyl oder ein pharmazeutisch annehmbares Kation bedeutet, Y Alkyl, Halogenalkyl, Polyhalogenalkyl mit 1 bis 3 Kohlenstoffatomen, Cyclopropyl, Hydroxyäthyl, Vinyl, Allyl, Phenyl, 4-Hydroxyphenyl oder 4-Fluorphenyl ist, $R_2$ Wasserstoff oder Fluor darstellt, $R_3$ Phenyl substituiert durch R' und R'' bedeutet, wobei R' Wasserstoff, Alkylsulfinyl mit 1 bis 4 Kohlenstoffatomen, Alkylsulfonyl mit 1 bis 4 Kohlenstoffatomen, Hydroxy, Alkoxy mit 1 bis 4 Kohlenstoffatomen, Hydroxyalkyl mit 1 bis 3 Kohlenstoffatomen, Amino, Aminoalkyl mit 1 bis 3 Kohlenstoffatomen, Formamido, Alkanoylamino mit 2 oder 3 Kohlenstoffatomen, Aminosulfonyl, Nitro, Formyl oder N-(N',N'-Dimethylformamidino) ist und R'' Wasserstoff, 3-Hydroxy oder 3-Chlor darstellt, welches umfaßt:

(a) Umsetzen einer Arylmetallverbindung mit einer Arylgruppe $R_3$, wie oben definiert, ausgenommen daß $R_3$ eine andere Bedeutung als Phenyl substituiert mit Alkanoylamino hat, mit einer Verbindung der Formel

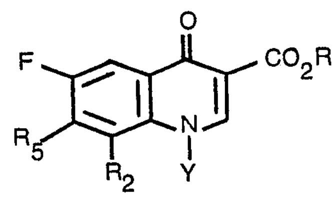

worin R Alkyl mit 1 bis 6 Kohlenstoffatomen oder Benzyl bedeutet, $R_2$ und Y wie oben definiert sind und $R_5$ Brom oder Jod bedeutet, in Anwesenheit eines Übergangsmetallkatalysators oder

(b) Umsetzen einer Verbindung der Formel

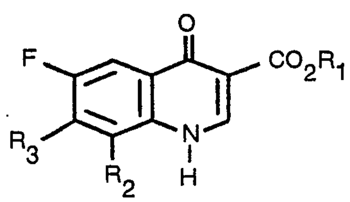

worin $R_1$, $R_2$ und $R_3$ wie oben definiert sind, ausgenommen daß $R_1$ eine andere Bedeutung als Wasserstoff hat, mit einer Verbindung der Formel Y—Hal, worin Y wie oben definiert ist und Hal Halogen bedeutet, und, wenn notwendig,

wenn $R_3$ Nitrophenyl ist, Nitrieren einer entsprechenden Verbindung, worin $R_3$ Phenyl ist,

wenn $R_3$ Aminophenyl ist, Reduzieren einer entsprechenden Verbindung, worin $R_3$ Nitrophenyl ist,

wenn $R_3$ Formamidophenyl ist, Umsetzen einer entsprechenden Verbindung, worin $R_3$ Aminophenyl ist, mit Ameisensäure oder einem Ameisensäurederivat,

wenn $R_3$ Alkanoylaminophenyl ist, Umsetzen einer entsprechenden Verbindung, worin $R_3$ Aminophenyl ist, mit einem eine Alkanoylgruppe enthaltenden Reagens,

wenn $R_3$ Aminosulfonylphenyl ist, Umsetzen einer entsprechenden Verbindung, worin $R_3$ Chlorsulfonylphenyl ist, mit einem Amino einführenden Reagens, wobei die Verbindung, worin $R_3$ Chlorsulfonylphenyl ist, durch Umsetzen einer entsprechenden Verbindung, worin $R_3$ Phenyl ist, mit Chlorsulfonsäure erhalten wird,

wenn $R_3$ Hydroxyphenyl ist, Umsetzen einer entsprechenden Verbindung, worin $R_3$ Methoxyphenyl ist, mit einem ätherspaltenden Reagens,

wenn $R_3$ Hydroxymethylphenyl ist, Umsetzen einer entsprechenden Verbindung, worin $R_3$ Trialkylsilyloxymethylphenyl ist, wobei jedes Alkyl 1 bis 4 Kohlenstoffatome aufweist, mit einem ätherspaltenden Reagens,

wenn $R_3$ Aminomethylphenyl ist, Hydrieren einer entsprechenden Verbindung, worin $R_3$ Azidomethylphenyl ist,

wenn $R_3$ Formylphenyl ist, Oxidieren einer entsprechenden Verbindung, worin $R_3$ Hydroxymethylphenyl ist,

wenn $R_3$ Alkylsulfinylphenyl oder Alkylsulfonylphenyl ist, Oxidieren einer entsprechenden Verbindung, worin $R_3$ Alkylmercaptophenyl ist, und

wenn $R_1$ Wasserstoff ist, Hydrolysieren einer entsprechenden Verbindung, worin $R_1$ Alkyl oder Benzyl ist, oder Hydrogenolysieren einer entsprechenden Verbindung, worin $R_1$ Benzyl ist.

2. Verfahren nach Anspruch 1, worin $R_1$ Wasserstoff, Y 4-Fluorphenyl, $R_2$ Wasserstoff und $R_3$ 4-Hydroxyphenyl bedeuten.

3. Verfahren, wie in Anspruch 1 beansprucht, worin die Reaktion (a) in einem Ätherlösungsmittel durchgeführt wird.

4. Verfahren, wie in Anspruch 3 beansprucht, worin dieses Ätherlösungsmittel ausgewählt wird aus Diäthyläther und Tetrahydrofuran.

5. Verfahren, wie in Anspruch 1 beansprucht, worin das Metall in der Arylmetallverbindung Magnesium, Zink, Cadmium, Zinn, Silber, Kupfer, Bor oder Aluminium ist.

6. Verfahren, wie in Anspruch 5 beansprucht, worin dieses Metall Zink ist.

7. Verfahren, wie in Anspruch 1 beansprucht, worin die Arylmetallverbindung ein Arylzinkhalogenid ist.

8. Verfahren, wie in Anspruch 1 beansprucht, worin der Übergangsmetallkatalysator im (O)- oder (II)-Oxidationszustand ist.

9. Verfahren, wie in Anspruch 8 beansprucht, worin das Übergangsmetall in diesem Katalysator Nickel, Palladium oder Platin ist.

10. Verfahren, wie in Anspruch 8 beansprucht, worin der Übergangsmetallkatalysator ein Komplex ist, der ein Übergangsmetall und einen phosphorhaltigen Liganden enthält.

11. Verfahren, wie in Anspruch 10 beansprucht, worin der Katalysator Tetrakis(triphenylphosphin)-palladium ist.

12. Verfahren, wie in Anspruch 10 beansprucht, worin der Katalysator Dichlorbis(triphenylphosphin)-nickel(II) ist.

13. Verbindung der Formel (I), wie in Anspruch 1 definiert.

**Revendications pour les Etats contractants: BE CH/LI DE FR IT LU NL SE GB**

1. Composé choisi parmi ceux qui répondent à la formule:

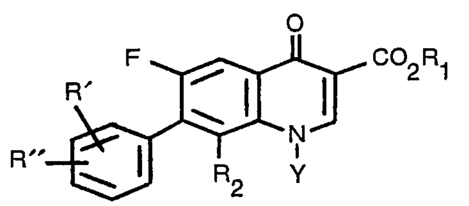

(I),

dans laquelle:

$R_1$ représente l'hydrogène, un groupe alkyle ayant de 1 à 6 atomes de carbone, benzyle ou un cation pharmaceutiquement acceptable;

$R_2$ représente l'hydrogène ou un radical fluoro;

Y est choisi parmi les radicaux alkyle, halogénoalkyle et polyhalogénoalkyle ayant de 1 à 3 atomes de carbone, hydroxyéthyle, cyclopropyle, vinyle, allyle, phényle, 4-hydroxyphényle et 4-fluorophényle;

R′ représente l'hydrogène, un groupe alkylsulfinyle ayant de 1 à 4 atomes de carbone, alkylsulfonyle ayant de 1 à 4 atomes de carbone, hydroxy, alcoxy ayant de 1 à 4 atomes de carbone, hydroxyalkyle ayant de 1 à 3 atomes de carbone, amino, aminoalkyle ayant de 1 à 3 atomes de carbone, formamido, alcanoylamino ayant 2 ou 3 atomes de carbone, aminosulfonyle, nitro, formyle ou N-(N′,N′-diméthylformamidino); et

R″ représente l'hydrogène, un radical 3-hydroxy ou 3-chloro.

2. Composé selon la revendication 1, caractérisé en ce que ledit composé est choisi parmi les suivants:

acide 6,8-difluoro-7-phényl-1-éthyl-1,4-dihydroquinol-4-one 3-carboxylique
acide 6,8-difluoro-7-(4-hydroxyphényl)-1-éthyl-1,4-dihydroquinol-4-one 3-carboxylique
acide 6,8-difluoro-7-(3-chloro-4-hydroxyphényl)-1-éthyl-1,4-dihydroquinol-4-one 3-carboxylique
acide 6,8-difluoro-7-(3-hydroxyphényl)-1-éthyl-1,4-dihydroquinol-4-one 3-carboxylique
acide 6,8-difluoro-7-(4-aminométhylphényl)-1-éthyl-1,4-dihydroquinol-4-one 3-carboxylique
acide 6,8-difluoro-7-(3-aminométhylphényl)-1-éthyl-1,4-dihydroquinol-4-one 3-carboxylique
acide 6,8-difluoro-7-(4-hydroxyméthylphényl)-1-éthyl-1,4-dihydroquinol-4-one 3-carboxylique
acide 6,8-difluoro-7-(3-hydroxyméthylphényl)-1-éthyl-1,4-dihydroquinol-4-one 3-carboxylique
acide 6,8-difluoro-7-(4-méthylsulfinylphényl)-1-éthyl-1,4-dihydroquinol-4-one 3-carboxylique
acide 6,8-difluoro-7-(3-méthylsulfonylphényl)-1-éthyl-1,4-dihydroquinol-4-one 3-carboxylique
acide 6,8-difluoro-7-(4-méthoxyphényl)-1-éthyl-1,4-dihydroquinol-4-one 3-carboxylique, et
acide 6,8-difluoro-7-(3-hydroxyméthyl-4-hydroxy)-1-éthyl-1,4-dihydroquinol-4-one 3-carboxylique

3. Composé selon la revendication 1, caractérisé en ce que ledit composé est choisi parmi les suivants:
acide 6-fluoro-7-phényl-1-éthyl-1,4-dihydroquinol-4-one 3-carboxylique
acide 6-fluoro-7-(4-aminophényl)-1-éthyl-1,4-dihydroquinol-4-one 3-carboxylique
acide 6-fluoro-7-(4-nitrophényl)-1-éthyl-1,4-dihydroquinol-4-one 3-carboxylique
acide 6-fluoro-7-(4-formamidophényl)-1-éthyl-1,4-dihydroquinol-4-one 3-carboxylique
acide 6-fluoro-7-(4-acétamidophényl)-1-éthyl-1,4-dihydroquinol-4-one 3-carboxylique
acide 6-fluoro-7-(4-N-(N',N'-diméthylformamidino)phényl-1-éthyl-1,4-dihydroquinol-4-one 3-carboxylique
acide 6-fluoro-7-(4-formylphényl)-1-éthyl-1,4-dihydroquinol-4-one 3-carboxylique
acide 6-fluoro-7-(4-hydroxyméthylphényl)-1-éthyl-1,4-dihydroquinol-4-one 3-carboxylique
acide 6-fluoro-7-(3-hydroxyméthylphényl)-1-éthyl-1,4-dihydroquinol-4-one 3-carboxylique, et
acide 6-fluoro-7-(4-aminosulfonylphényl)-1-éthyl-1,4-dihydroquinol-4-one 3-carboxylique.

4. Composé selon la revendication 1, caractérisé en ce que ledit composé est l'acide 6-fluoro-7-(4-hydroxyphényl)-1-(4-fluorophényl)-1,4-dihydroquinol-4-one 3-carboxylique.

5. Composition anti-bactérienne, caractérisée en ce qu'elle renferme un composé selon l'une quelconque des revendications 1 à 4 et un véhicule pharmaceutiquement acceptable.

6. Composé selon l'une quelconque des revendications 1 à 4 ou un sel d'addition pharmaceutiquement acceptable d'un tel composé, en vue de son utilisation en médecine.

7. Utilisation d'un composé selon l'une quelconque des revendications 1 à 4 ou d'un sel d'addition pharmaceutiquement acceptable d'un tel composé pour la fabrication d'un médicament anti-bactérien.

**Revendications pour l'Etat contractant: AT**

1. Procédé de préparation d'arylquinolones 7-substituées de formule:

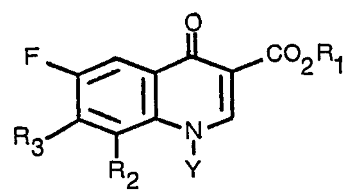

(I),

dans laquelle:

$R_1$ représente l'hydrogène, un groupe alkyle ayant de 1 à 6 atomes de carbone ou benzyle ou un cation pharmaceutiquement acceptable;

Y représente un groupe alkyle, halogénoalkyle, polyhalogénoalkyle ayant de 1 à 3 atomes de carbone, cyclopropyle, hydroxyéthyle, vinyle, allyle, phényle, 4-hydroxyphényle ou 4-fluorophényle;

$R_2$ représente l'hydrogène ou un radical fluoro;

$R_3$ représente un radical phényle substitué par R' et R''; et

R' représente l'hydrogène, un radical alkylsulfinyle ayant de 1 à 4 atomes de carbone, alkylsulfonyle ayant de 1 à 4 atomes de carbone, hydroxy, alcoxy ayant de 1 à 4 atomes de carbone, hydroxyalkyle ayant de 1 à 3 atomes de carbone, amino, aminoalkyle ayant de 1 à 3 atomes de carbone, formamido, alcanoylamino ayant 2 ou 3 atomes de carbone, aminosulfonyle, nitro, formyle ou N-(N',N'-diméthylformamidino); et

R'' représente l'hydrogène ou un groupe 3-hydroxy ou 3-chloro

qui consiste:

(a) à faire réagir un composé aryle métallique contenant un groupe aryle $R_3$ tel que défini ci-dessus, excepté que $R_3$ ne représente pas un radical phényle substitué par un groupe alcanoylamino avec un composé de formule:

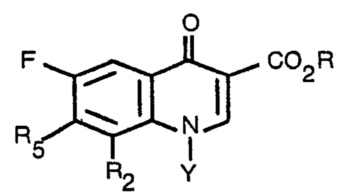

dans laquelle

R est un groupe alkyle ayant de 1 à 6 atomes de carbone ou un groupe benzyle;

$R_2$ et Y sont tels que définis ci-dessus et $R_5$ est un radical bromo ou iodo, en présence d'un catalyseur à base d'un métal de transition, ou

(b) à faire réagir un composé de formule:

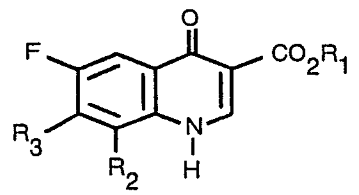

dans laquelle $R_1$, $R_2$ et $R_3$ sont tels que définis ci-dessus, excepté que $R_1$ ne représente pas l'hydrogène, avec un composé de formule Y—Hal dans laquelle Y est tel que défini ci-dessus et Hal représente l'hydrogène; et, si nécessaire,

lorsque $R_3$ est un groupe nitrophényle, à nitrer un composé correspondant dans lequelle $R_3$ représente un groupe phényle;

lorsque $R_3$ est un groupe aminophényle, à réduire un composé correspondant dans lequel $R_3$ est un groupe nitrophényle;

lorsque $R_3$ est un groupe formamidophényle, à faire réagir un composé correspondant dans lequel $R_3$ est un groupe aminophényle avec l'acide formique ou un dérivé d'acide formique;

lorsque $R_3$ est un radical alcanoylaminophényle, à faire réagir un composé correspondant dans lequel $R_3$ est un groupe aminophényle avec un réactif contenant un groupe alcanoyle;

lorsque $R_3$ est un groupe aminosulfonylphényle, à faire réagir un composé correspondant dans lequel $R_3$ est un groupe chlorosulfonylphényle avec un réactif introducteur de groupe amino; ledit composé dans lequel $R_3$ est un groupe chlorosulfonylphényle étant obtenu par réaction d'un composé correspondant dans lequel $R_3$ est un groupe phényle avec l'acide chlorosulfonique;

lorsque $R_3$ est un groupe hydroxyphényle, à faire réagir un composé correspondant dans lequel $R_3$ est un groupe méthoxyphényle avec un réactif clivant la fonction éther;

lorsque $R_3$ est un groupe hydroxyméthylphényle, à faire réagir un composé correspondant dans lequel $R_3$ est un groupe trialkylsilyloxyméthylphényle dans lequel chaque groupe alkyle comporte de 1 à 4 atomes de carbone avec un réactif clivant la fonction éther;

lorsque $R_3$ est un groupe aminométhylphényle, à hydrogéner un composé correspondant dans lequel $R_3$ est un groupe azidométhylphényle;

lorsque $R_3$ est un groupe formylphényle, à oxyder un composé correspondant dans lequel $R_3$ est un groupe hydroxyméthylphényle;

lorsque $R_3$ est un groupe alkylsulfinylphényle ou alkylsulfonylphényle, à oxyder un composé correspondant dans lequel $R_3$ est un groupe alkylmercaptophényle; et

lorsque $R_1$ représente l'hydrogène, à hydrolyser un composé correspondant dans lequel $R_1$ représente un groupe alkyle ou benzyle, ou à hydrogénolyser un composé correspondant dans lequel $R_1$ est un groupe benzyle.

2. Procédé selon la revendication 1, dans lequel $R_1$ représente l'hydrogène, Y un groupe 4-fluorophényle, $R_2$ l'hydrogène et $R_3$ un groupe 4-hydroxyphényle.

3. Procédé selon la revendication 1, dans lequel ladite réaction (a) est mise en oeuvre dans un solvant éther.

4. Procédé selon la revendication 3, dans lequel ledit solvant éther est choisi entre le diéthyléther et le tétrahydrofurane.

5. Procédé selon la revendication 1, dans lequel le métal dudit composé arylmétallique est le magnésium, le zinc, le cadmium, l'étain, l'argent, le cuivre, le bore ou l'aluminium.

6. Procédé selon la revendication 5, dans lequel ledit métal est le zinc.

7. Procédé selon la revendication 1, dans lequel ledit composé arylmétallique est un halogénure d'arylzinc.

8. Procédé selon la revendication 1, dans lequel ledit catalyseur à base de métal de transition est à l'état d'oxydation (O) ou (II).

9. Procédé selon la revendication 8, dans lequel ledit métal de transition compris dans ledit catalyseur est le nickel, le palladium ou le platine.

10. Procédé selon la revendication 8, dans lequel ledit catalyseur à base de métal de transition est un complexe contenant un métal de transition et un groupe de coordination contenant du phosphore.

11. Procédé selon la revendication 10, dans lequel ledit catalyseur est le tétrakis(triphénylphosphine)-palladium.

12. Procédé selon la revendication 10, dans lequel ledit catalyseur est le dichlorobis(triphénylphosphine) nickel (II).

13. Composé de formule (I) tel que défini dans la revendication 1.